(19) Europäisches Patentamt
European Patent Office
Office européen des brevets

(11) EP 3 503 240 B1

(12) **EUROPEAN PATENT SPECIFICATION**

(45) Date of publication and mention of the grant of the patent:
**20.11.2024 Bulletin 2024/47**

(21) Application number: **17209694.3**

(22) Date of filing: **21.12.2017**

(51) International Patent Classification (IPC):
***H10K 85/60*** *(2023.01)* ***C07D 405/04*** *(2006.01)*
***C07D 201/00*** *(2006.01)* ***C07D 409/04*** *(2006.01)*
***C07D 421/04*** *(2006.01)* *H10K 50/165* *(2023.01)*

(52) Cooperative Patent Classification (CPC):
**C07D 405/04; C07D 409/04; C07D 421/04; H10K 85/654; H10K 85/6574;** C07D 201/00; H10K 50/165; Y02E 10/549

(54) **ORGANIC SEMICONDUCTOR LAYER**

ORGANISCHE HALBLEITERSCHICHT

COUCHE À SEMI-CONDUCTEUR ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.06.2019 Bulletin 2019/26**

(73) Proprietors:
- **Novaled GmbH**
  **01099 Dresden (DE)**
- **Samsung SDI Co., Ltd.**
  **Yongin-si, Gyeonggi-do 17084 (KR)**

(72) Inventors:
- **Schulze, Benjamin**
  **01099 Dresden (DE)**
- **Rothe, Carsten**
  **01099 Dresden (DE)**
- **Jang, Kipo**
  **Suwon-si, Gyeonggi-do, 16678 (KR)**
- **Lui, Jinhyun**
  **Suwon-si, Gyeonggi-do, 16678 (KR)**

(74) Representative: **Michalski Hüttermann & Partner Patentanwälte mbB**
**Kaistraße 16A**
**40221 Düsseldorf (DE)**

(56) References cited:
**WO-A1-2015/000549**
**CN-A- 105 153 130**
**US-A1- 2013 248 830**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 503 240 B1

## Description

## Technical Field

The present invention relates to an organic semiconductor layer, in particular to organic semiconductor layer comprising a compound substituted with bulky groups and a dopant, suitable for use as an organic semiconductor layer for electronic devices, and a method of manufacturing the same.

## Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EMI,, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

CN105153130 A provides an organic light-emitting compound adopting a triazine derivative shown in the structural formula I. The compound has better thermal stability, high luminous efficiency and high luminous purity, can be used for manufacturing OLEDs (organic light-emitting devices) and can be applied to the fields of organic solar cells, organic thin-film transistors or organic photoreceptors. The invention further provides an OLED which comprises an anode, a cathode and an organic layer, wherein the organic layer comprises one or more of a luminous layer, a hole injection layer, a hole transporting layer, a hole blocking layer, an electron injection layer and an electron transporting layer, and at least one layer in the organic layer contains the compound shown in the structural formula below:

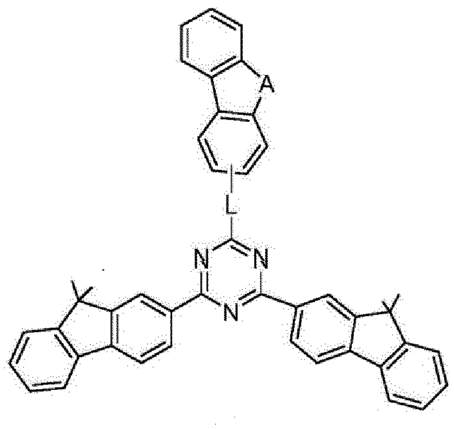

wherein A = 0 or S.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic electronic device, such as an organic light emitting diode, may be applied to a large-size flat panel display.

Further, development of an organic semiconductor layer being capable to have an extended life span at higher current density and thereby at higher brightness is needed.

There remains a need to improve performance of organic semiconductor layers, organic semiconductor materials, as well as organic electronic devices thereof, in particular to achieve increased lifetime at higher current density and have a higher efficiency through improving the characteristics of the compounds comprised therein.

There is a need for alternative organic semiconductor materials and organic semiconductor layers as well as organic electronic devices having increased lifetime at higher current density, and/or improved efficiency at low operating voltage.

In particular there is a need for alternative compounds having increased lifetime at higher current density as well as improved efficiency, and at the same time keeping the operating voltage and thereby the power consumption low to deliver long battery life for example mobile electronic devices.

## DISCLOSURE

An aspect of the present invention provides in accordance with claim 1 (first embodiment) an organic semiconductor layer comprising:

- a compound of formula 1:

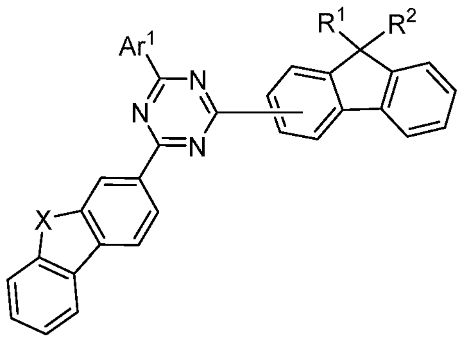

(1),

wherein

X is O, S or Se;
$Ar^1$ is selected from unsubstituted $C_6$ to $C_{24}$ aryl or unsubstituted $C_3$ to $C_{36}$ heteroaryl;
$R^1$, $R^2$ are independently selected from substituted or unsubstituted $C_6$ to $C_{24}$ aryl, substituted or unsubstituted $C_3$ to $C_{24}$ heteroaryl, or $R^1$ and $R^2$ are selected from substituted or unsubstituted $C_6$ to $C_{24}$ aryl and substituted or unsubstituted $C_3$ to $C_{24}$ heteroaryl and linked with each other via a single bond such that a five-membered ring is formed,
wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl;

and

- a dopant selected from an organic metal complex or metal halide.

Hetero atoms, if not otherwise stated, can be individually selected from N, O, S, B, Si, P, Se, preferably from N, O and S and more preferred is N.

If not otherwise stated H can represent hydrogen or deuterium.

An aspect of the present invention provides in accordance with claim 10 (second embodiment) an organic semiconductor layer comprising:

- a compound of formula 1:

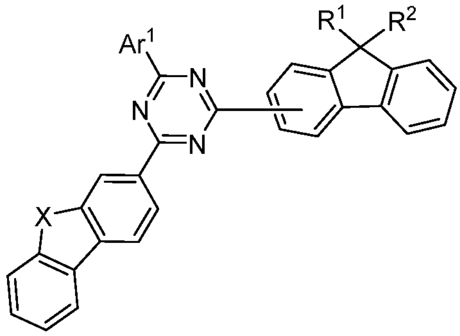

(1),

wherein

| | |
|---|---|
| X | is O, S or Se; |
| $Ar^1$ | is selected from $C_1$ to $C_{16}$ alkyl, substituted or unsubstituted $C_6$ to $C_{40}$ aryl, substituted or unsubstituted $C_3$ to $C_{40}$ heteroaryl, wherein<br>the substituents of the substituted $C_6$ to $C_{40}$ aryl and substituted $C_3$ to $C_{40}$ heteroaryl are selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perdeuterated $C_1$ to $C_{16}$ alkoxy, $C_6$ to $C_{24}$ aryl, $C_3$ to $C_{25}$ heteroaryl, - $PXR^3R^4$, D, F or CN, wherein<br>$R^3$, $R^4$ are independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl;<br>wherein the substituted $C_6$ to $C_{40}$ aryl and/or substituted $C_3$ to $C_{40}$ heteroaryl of $Ar^1$ comprises at least one - $PXR^3R^4$ substituent; |
| R1, R2 | are independently selected from substituted or unsubstituted $C_6$ to $C_{24}$ aryl, substituted or unsubstituted $C_3$ to $C_{24}$ heteroaryl, or $R^1$ and $R^2$ are selected from substituted or unsubstituted $C_6$ to $C_{24}$ aryl and substituted or unsubstituted $C_3$ to $C_{24}$ heteroaryl and linked with each other via a single bond such that a five-membered ring is formed,<br>wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl; |

and

- a dopant selected from an organic metal complex or metal halide.

According to the second embodiment, the organic semiconductor layer may comprise:

- a compound of formula 1:

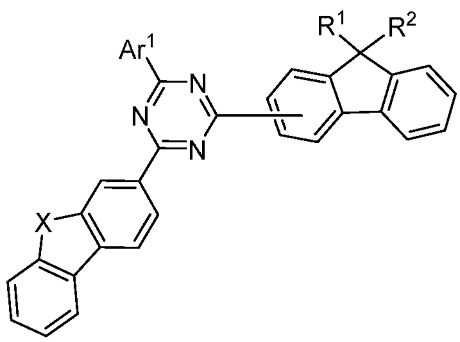

(1),

wherein

X is O, S or Se;

$Ar^1$ is selected from $C_1$ to $C_{16}$ alkyl, substituted or unsubstituted $C_6$ to $C_{40}$ aryl, substituted or unsubstituted $C_3$ to $C_{40}$ heteroaryl, wherein
the substituents of the substituted $C_6$ to $C_{40}$ aryl and substituted $C_3$ to $C_{40}$ heteroaryl are selected from -$PXR^3R^4$, wherein
$R^3$, $R^4$ are independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl;

$R^1$, $R^2$ are independently selected from substituted or unsubstituted $C_6$ to $C_{24}$ aryl, substituted or unsubstituted $C_3$ to $C_{24}$ heteroaryl, or $R^1$ and $R^2$ are selected from substituted or unsubstituted $C_6$ to $C_{24}$ aryl and substituted or unsubstituted $C_3$ to $C_{24}$ heteroaryl and linked with each other via a single bond such that a five-membered ring is formed,
wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl;

and

- a dopant selected from an organic metal complex or metal halide.

According to the second embodiment, the organic semiconductor layer may comprise:

- a compound of formula 1:

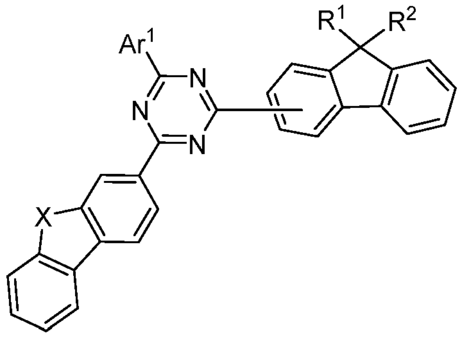

(1),

wherein

X is O, S or Se;

$Ar^1$ is selected from $C_1$ to $C_{16}$ alkyl, substituted or unsubstituted $C_6$ to $C_{40}$ aryl, substituted or unsubstituted $C_3$ to $C_{40}$ heteroaryl, wherein
the substituents of the substituted $C_6$ to $C_{40}$ aryl and substituted $C_3$ to $C_{40}$ heteroaryl are selected from -$PXR^3R^4$, wherein

$R^3$, $R^4$ are independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl;

$R^1$, $R^2$ are independently selected from substituted or unsubstituted $C_6$ to $C_{24}$ aryl, substituted or unsubstituted $C_3$ to $C_{24}$ heteroaryl,
wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$

to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl;

and

- a dopant selected from an organic metal complex or metal halide.

According to the first embodiment, the organic semiconductor layer may comprise:

- a compound of formula 1:

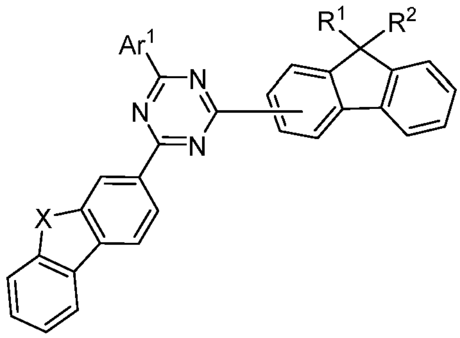

(1),

wherein

X is O, S or Se;
$Ar^1$ is selected from unsubstituted $C_6$ to $C_{24}$ aryl or unsubstituted $C_3$ to $C_{36}$ heteroaryl;
$R^1$, $R^2$ are independently selected from unsubstituted $C_6$ to $C_{24}$ aryl, unsubstituted $C_3$ to $C_{24}$ heteroaryl, or $R^1$ and $R^2$ are selected from unsubstituted $C_6$ to $C_{24}$ aryl and unsubstituted $C_3$ to $C_{24}$ heteroaryl and linked with each other via a single bond such that a five-membered ring is formed;

and

- a dopant selected from an organic metal complex or metal halide.

According to the first embodiment, the organic semiconductor layer may comprises:

- a compound of formula 1:

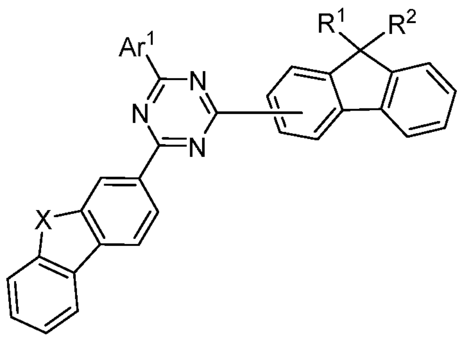

(1),

wherein

X is O, S or Se;
$Ar^1$ is selected from unsubstituted $C_6$ to $C_{24}$ aryl or unsubstituted $C_3$ to $C_{36}$ heteroaryl;
$R^1$, $R^2$ are independently selected from unsubstituted $C_6$ to $C_{24}$ aryl, unsubstituted $C_3$ to $C_{24}$ heteroaryl;

and

- a dopant selected from an organic metal complex or metal halide.

According to the second embodiment, the organic semiconductor layer may comprise:

- a compound of formula 1:

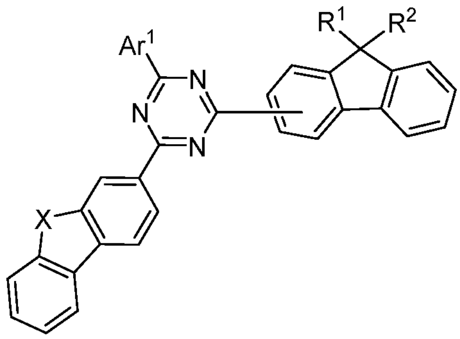

(1),

wherein

X is O, S or Se;
$Ar^1$ is selected from substituted $C_6$ to $C_{40}$ aryl, substituted $C_3$ to $C_{40}$ heteroaryl, wherein the substituents of the substituted $C_6$ to $C_{40}$ aryl and substituted $C_3$ to $C_{40}$ heteroaryl is - $PXR^3R^4$, wherein
$R^3$, $R^4$ are independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl;
$R^1$, $R^2$ are independently selected from substituted $C_6$ to $C_{24}$ aryl, substituted $C_3$ to $C_{24}$ heteroaryl, wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl;

and
- a dopant selected from an organic metal complex or metal halide.

According to another embodiment of the compound of formula 1, wherein X may be selected from O or S.
According to another embodiment of the compound of formula 1, wherein X is O.
According to another embodiment of the compound of formula 1, wherein

$R^1$, $R^2$ are independently selected from substituted or unsubstituted $C_6$ to $C_{24}$ aryl, substituted or unsubstituted $C_3$ to $C_{24}$ heteroaryl,
wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl.

According to another embodiment of the compound of formula 1, wherein

$R^1$, $R^2$ are independently selected from substituted or unsubstituted $C_6$ to $C_{18}$ aryl, substituted or unsubstituted $C_3$ to $C_{17}$ heteroaryl,
wherein the substituents of the substituted $C_6$ to $C_{18}$ aryl and substituted $C_3$ to $C_{17}$ heteroarylene are selected from H, D, $C_1$ to $C_6$ alkyl, partially or perfluorinated $C_1$ to $C_6$ alkyl, partially or perdeuterated $C_1$ to $C_6$ alkyl, $C_1$ to $C_6$ alkoxy, $C_1$ to $C_6$ thioalkyl, $C_3$ to $C_6$ branched alkyl, $C_3$ to $C_6$ cyclic alkyl, $C_3$ to $C_6$ branched alkoxy, $C_3$ to $C_6$ cyclic alkoxy, $C_3$ to $C_6$ branched thioalkyl, $C_3$ to $C_6$ cyclic thioalkyl, $C_6$ to $C_{18}$ aryl and $C_3$ to $C_{17}$ heteroaryl.

According to another embodiment of the compound of formula 1, wherein

$R^1$, $R^2$ are independently selected from substituted or unsubstituted $C_6$ to $C_{18}$ aryl, wherein the substituents of the substituted $C_6$ to $C_{18}$ aryl are selected from H, D, $C_1$ to $C_6$ alkyl, partially or perfluorinated $C_1$ to $C_6$ alkyl, partially or perdeuterated $C_1$ to $C_6$ alkyl, $C_1$ to $C_6$ alkoxy, preferably $R^1$, $R^2$ are independently selected from phenyl, biphenyl, terphenyl, naphthyl, phenanthrenyl, also preferred $R^1$, $R^2$ are selected from phenyl.

According to another embodiment of the compound of formula 1, wherein

$R^1$, $R^2$ are independently selected from substituted or unsubstituted $C_3$ to $C_{17}$ heteroaryl, wherein the substituents of the substituted $C_3$ to $C_{17}$ heteroaryl are selected from H, D, $C_1$ to $C_6$ alkyl, partially or perfluorinated $C_1$ to $C_6$ alkyl, partially or perdeuterated $C_1$ to $C_6$ alkyl, $C_1$ to $C_6$ alkoxy, preferably $R^1$, $R^2$ are independently selected from pyridyl, quinolinyl, quinazolinyl.

According to another embodiment of the compound of formula 1, wherein $R^1$, $R^2$ are independently selected from unsubstituted unsubstituted $C_6$ to $C_{24}$ aryl, or unsubstituted $C_3$ to $C_{24}$ heteroaryl, preferably $R^1$, $R^2$ are independently selected from phenyl, biphenyl, terphenyl naphthyl, phenanthrenyl, pyridyl, quinolinyl, quinazolinyl, more preferred $R^1$ is selected from phenyl, biphenyl, terphenyl naphthyl, phenanthrenyl and $R^2$ is selected from pyridyl, quinolinyl, quinazolinyl.

A high Tg may be achieved when $R^1$, $R^2$ are selected in this range.

According to the first embodiment
$Ar^1$ is selected from substituted or unsubstituted $C_6$ to $C_{24}$ aryl or substituted or unsubstituted $C_3$ to $C_{36}$ heteroaryl, wherein the substituents of the substituted $C_6$ to $C_{24}$ aryl and substituted $C_3$ to $C_{36}$ heteroaryl may be selected from $C_1$ to $C_{12}$ alkyl, $C_1$ to $C_{12}$ alkoxy, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perfluorinated $C_1$ to $C_{12}$ alkyl, partially or perfluorinated $C_1$ to $C_{16}$ alkoxy, partially or perdeuterated $C_1$ to $C_{12}$ alkyl, partially or perdeuterated $C_1$ to $C_{16}$ alkoxy, $C_6$ to $C_{24}$ aryl, $C_3$ to $C_{25}$ heteroaryl, D, F or CN, preferably from $C_1$ to $C_{12}$ alkyl.

According to a preferred embodiment of the first embodiment,
$Ar^1$ may be selected from substituted or unsubstituted $C_6$ to $C_{18}$ aryl or substituted or unsubstituted $C_3$ to $C_{17}$ heteroaryl, wherein
the substituents of the substituted $C_6$ to $C_{18}$ aryl and substituted $C_3$ to $C_{17}$ heteroaryl may be selected from F or CN.

According to a preferred embodiment of the second embodiment,

$Ar^1$ may be selected from substituted or unsubstituted $C_6$ to $C_{18}$ aryl or substituted or unsubstituted $C_3$ to $C_{17}$ heteroaryl, wherein
the substituents of the substituted $C_6$ to $C_{18}$ aryl and substituted $C_3$ to $C_{17}$ heteroaryl may be selected from - $PXR^3R^4$, F or CN;
wherein at least one substituent of the substituted $C_6$ to $C_{18}$ aryl and substituted $C_3$ to $C_{17}$ heteroaryl is - $PXR^3R^4$.

According to another embodiment of the compound of formula 1, wherein
$Ar^1$ may be selected from unsubstituted $C_6$ to $C_{24}$ aryl, preferably a $C_6$ or $C_{12}$ aryl.

According to another embodiment of the compound of formula 1, wherein
$Ar^1$ may be selected from unsubstituted $C_6$ to $C_{12}$ aryl, or unsubstituted $C_3$ to $C_{18}$ heteroaryl, preferably phenyl or biphenyl and in addition preferred phenyl.

In another embodiment, $Ar^1$ may be selected from B1 to B6 and B16 to B23, preferably from B1 to B6, B16 to B 17 and B19.

According to another embodiment of the compound of formula 1, wherein $Ar^1$ may be independently selected from B1 to B 65 and B71 to B77, wherein

a) B1 to B6 are substituted or unsubstituted non-heteroaryl groups:

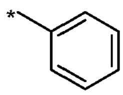 (B1), 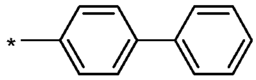 (B2), 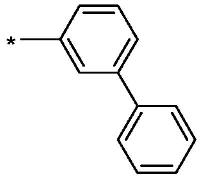 (B3), 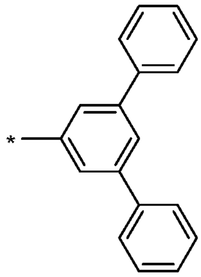 (B4),

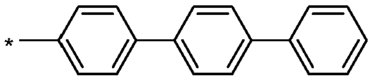 (B5), 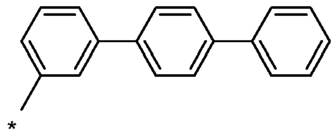 (B6);

or

b) B7 to B23 are substituted or unsubstituted annelated non-heteroaryl groups:

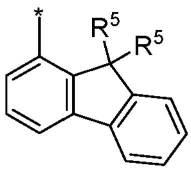 (B7), 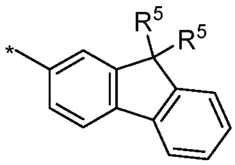 (B8), 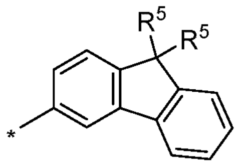 (B9), 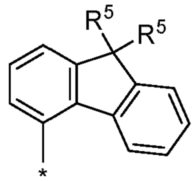 (B10), 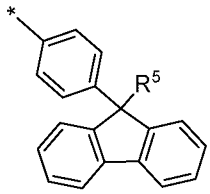 (B11),

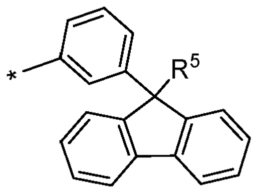 (B12), 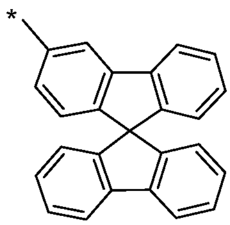 (B13), 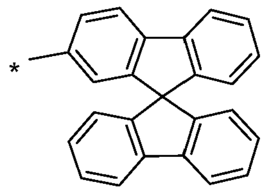 (B14), 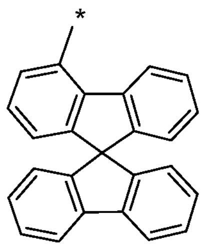 (B15),

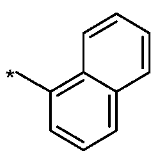 (B16), 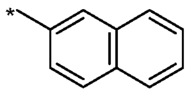 (B17), 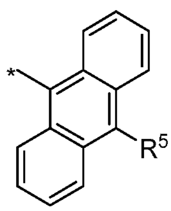 (B18), 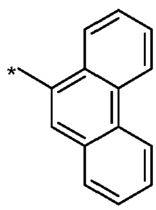 (B19), 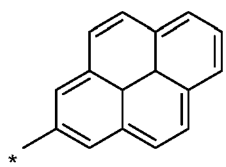 (B20),

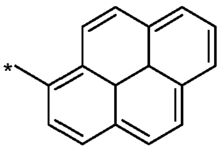
(B21),
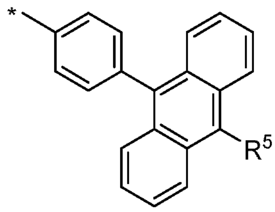
(B22),
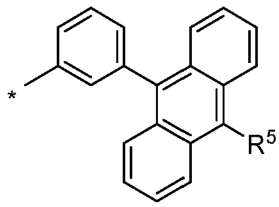
(B23);

or

c) B24 to B31 are dibenzofurane/dibenzothiophene group:

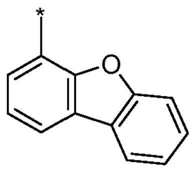
(B24),
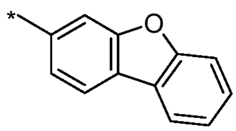
(B25),
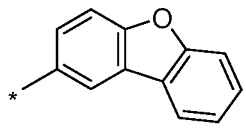
(B26),
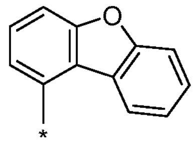
(B27),

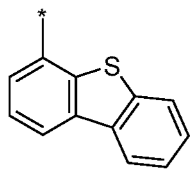
(B28),
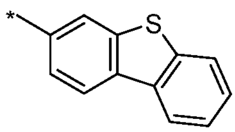
(B29),
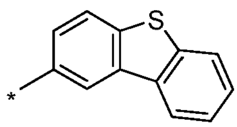
(B30),
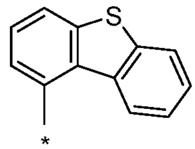
(B31);

or

d) B32 to B34 are unsubstituted pyridine groups:

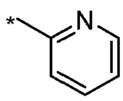
(B32),
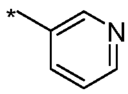
(B33),
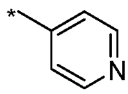
(B34);

or

e) B35 to B62 are unsubstituted or substituted hetero arylene groups:

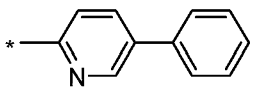
(B35),
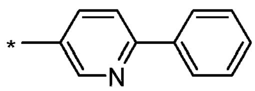
(B36),

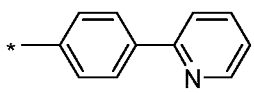
(B37),
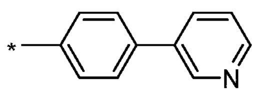
(B38),
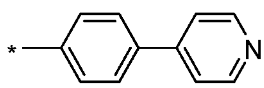
(B39),

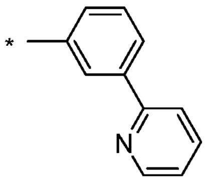 (B40), 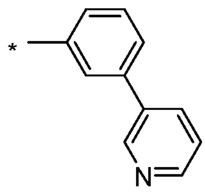 (B41), 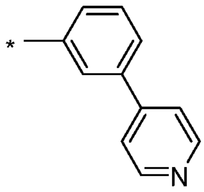 (B42), 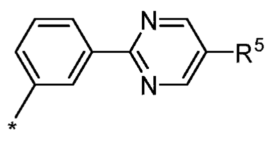 (B43),

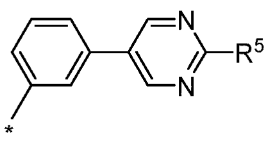 (B44), 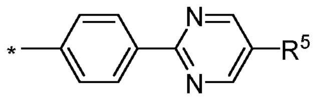 (B45), 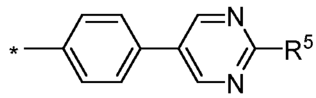 (B46),

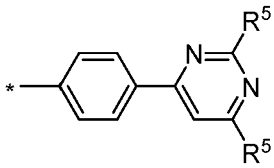 (B47), 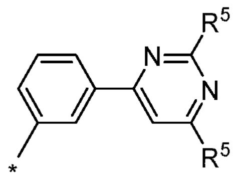 (B48), 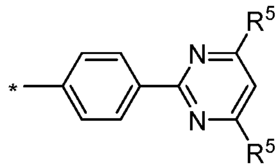 (B49),

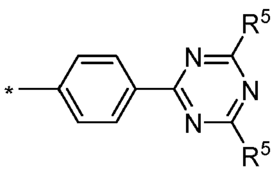 (B50), 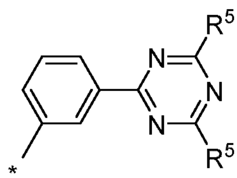 (B51), 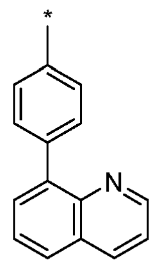 (B52),

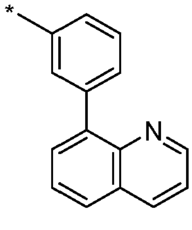 (B53), 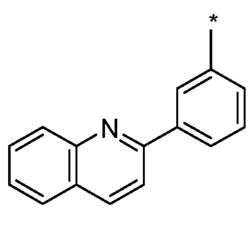 (B54), 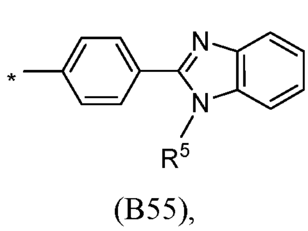 (B55),

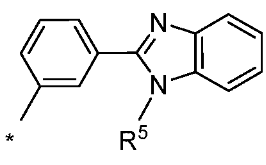 (B56), 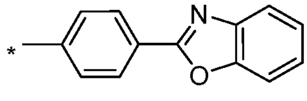 (B57), 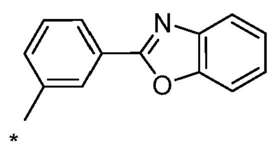 (B58),

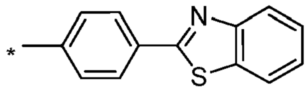
(B59),
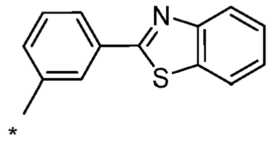
(B60),
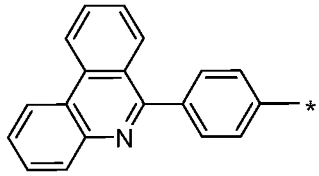
(B61),

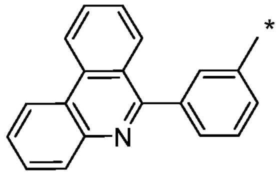
(B62);

or

f) B63 to B65 unsubstituted annelated hetero arylene groups:

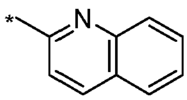
(B63),
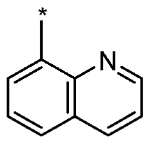
(B64)
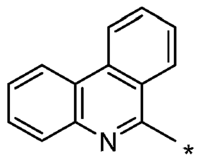
(B65);

or

i) B71 to B77 are carbazole groups

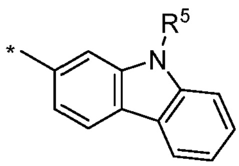
(B71),
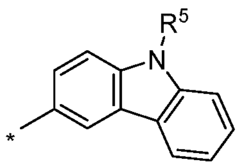
(B72),
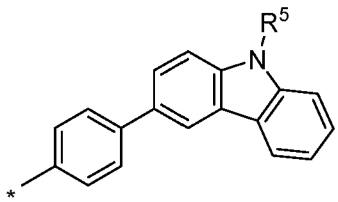
(B73),

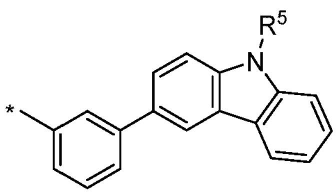
(B74),
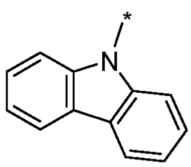
(B75),
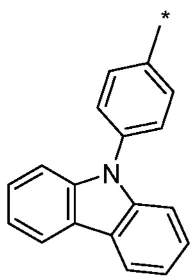
(B76),
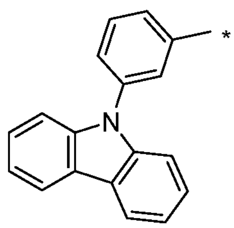
(B77);

wherein

the substituent $R^5$ is H.

In another embodiment, $Ar^1$ may be selected from B1 to B6 and B16 to B23, preferably from B 1 to B6, B16 to

B 17 and B 19.

According to another embodiment of the compound of formula 1, the compound may be selected from D1 to D8:

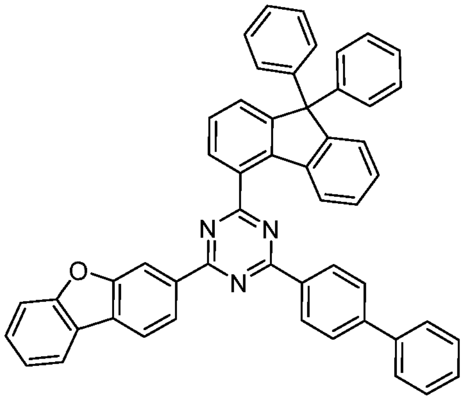

(D1),

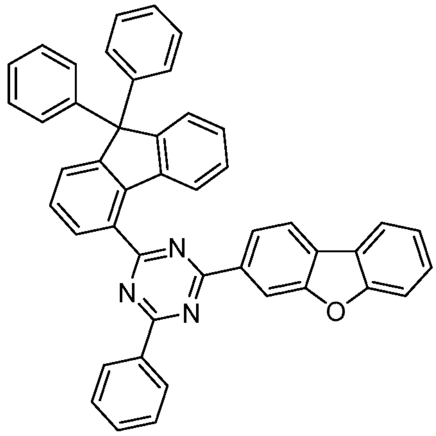

(D2),

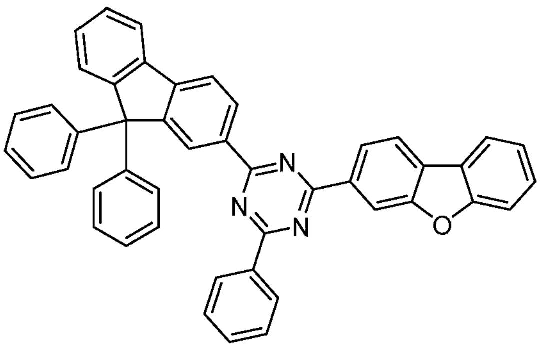

(D3),

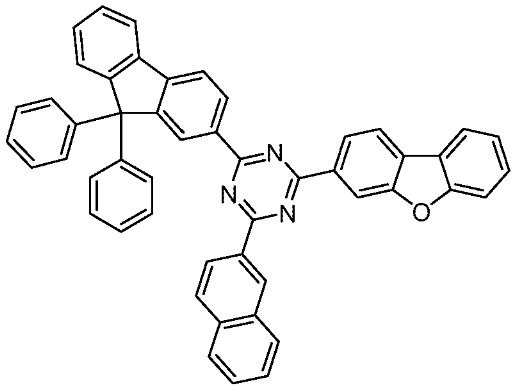

(D4),

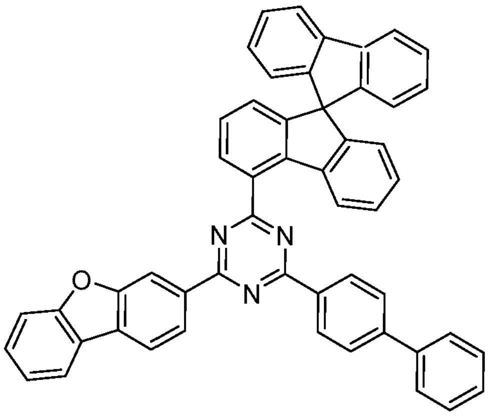

(D5),

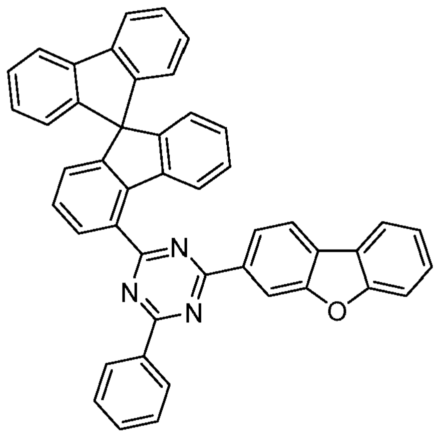

(D6),

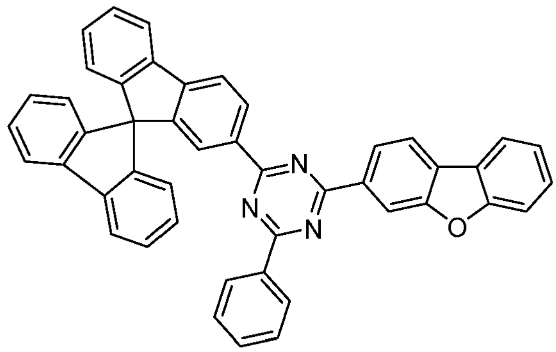

(D7),

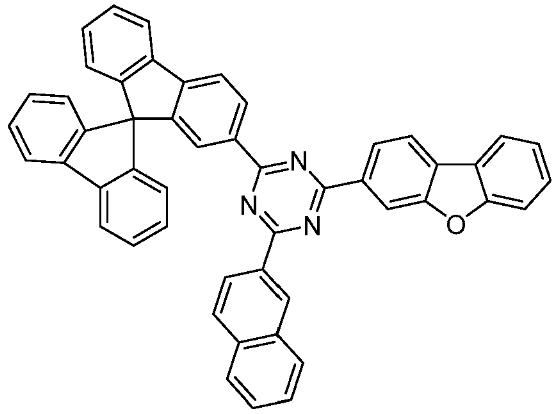

(D8).

According to another embodiment of the compound of formula 1, wherein $Ar^1$ may be independently selected from structures C1 to C5:

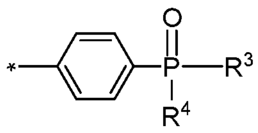

(C1),

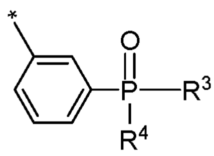

(C2),

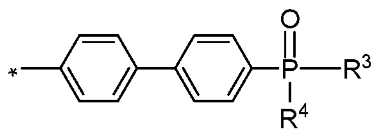

(C3),

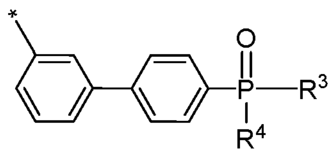

(C4),

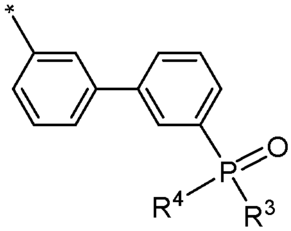

(C5),

wherein

$R^3$, $R^4$ may be independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl.

According to another embodiment of the compound of formula 1, wherein $R^3$, $R^4$ may be independently selected from $C_1$ to $C_8$ alkyl, $C_1$ to $C_8$ alkoxy, partially or perfluorinated $C_1$ to $C_8$ alkyl, partially or perfluorinated $C_1$ to $C_8$ alkoxy, partially or perdeuterated $C_1$ to $C_8$ alkyl, partially or perdeuterated $C_1$ to $C_8$ alkoxy, $C_6$ to $C_{12}$ aryl, $C_3$ to $C_{20}$ heteroaryl.

According to another embodiment of the compound of formula 1, wherein $R^3$, $R^4$ may be independently selected from $C_1$ to $C_8$ alkyl, partially or perdeuterated $C_1$ to $C_8$ alkyl, partially or perdeuterated $C_1$ to $C_8$ alkoxy, $C_6$ to $C_{12}$ aryl, $C_3$ to $C_{20}$ heteroaryl.

According to another embodiment of the compound of formula 1, wherein $R^3$, $R^4$ may be phenyl or $C_1$ to C4

alkyl, even more preferred phenyl or methyl.

According to another embodiment of the compound of formula 1, wherein $R^3$ and $R^4$ are selected the same.

According to an aspect the organic semiconductor layer can be used as a layer for an organic electronic device. For example, the organic electronic device can be an OLED or there like.

The organic semiconductor layer comprising a compound of formula 1 and a dopant selected from an organic metal complex or metal halide have strong electron transport characteristics to increase charge mobility and/or stability and thereby to improve luminance efficiency, voltage characteristics, and/or lifetime characteristics.

The organic semiconductor layer comprising a compound of formula 1 and a dopant selected from an organic metal complex or metal halide have high electron mobility and a low operating voltage.

The organic semiconductor layer comprising a compound of formula 1 and a dopant selected from an organic metal complex or metal halide may be non-emissive.

In the context of the present specification the term "essentially non-emissive" or "non-emitting" means that the contribution of the compound of formula 1 or layer comprising the compound of formula 1 and the dopant to the visible emission spectrum from the device is less than 10 %, preferably less than 5 %, further preferred less than 1 %, relative to the visible emission spectrum. The visible emission spectrum is an emission spectrum with a wavelength of $\geq$ 380 nm to $\leq$ 780 nm. Preferably, the organic semiconductor layer comprising the compound of formula 1 is essentially non-emissive or non-emitting.

The term "free of', "does not contain", "does not comprise" does not exclude impurities which may be present in the compounds prior to deposition. Impurities have no technical effect with respect to the object achieved by the present invention.

The operating voltage, also named U, is measured in Volt (V) at 10 milliAmpere per square centimeter (mA/cm2).

The candela per Ampere efficiency, also named cd/A efficiency, is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color.

The highest occupied molecular orbital, also named HOMO, and lowest unoccupied molecular orbital, also named LUMO, are measured in electron volt (eV).

The rate onset temperature is measured in °C and describes the VTE source temperature at which measurable evaporation of a compound commences at a pressure of less than $10^{-5}$ mbar.

The term "OLED", "organic light emitting diode", "organic light emitting device", "organic optoelectronic device" and "organic light-emitting diode" are simultaneously used and have the same meaning.

The term "transition metal" means and comprises any element in the d-block of the periodic table, which comprises groups 3 to 12 elements on the periodic table.

The term "group III to VI metal" means and comprises any metal in groups III to VI of the periodic table.

As used herein, "weight percent", "wt.-%", "percent by weight", "% by weight", and variations thereof refer to a composition, component, substance or agent as the weight of that composition, component, substance or agent of the respective electron transport layer divided by the total weight of the composition thereof and multiplied by 100. It is understood that the total weight percent amount of all components, substances or agents of the respective electron transport layer are selected such that it does not exceed 100 wt.-%.

As used herein, "volume percent", "vol.-%", "percent by volume", "% by volume", and variations thereof refer to an elemental metal, a composition, component, substance or agent as the volume of that elemental metal, component, substance or agent of the respective electron transport layer divided by the total volume of the respective electron transport layer thereof and multiplied by 100. It is understood that the total volume percent amount of all elemental metal, components, substances or agents of the respective cathode electrode layer are selected such that it does not exceed 100 vol.-%.

All numeric values are herein assumed to be modified by the term "about", whether or not explicitly indicated. As used herein, the term "about" refers to variation in the numerical quantity that can occur.

Whether or not modified by the term "about", the claims include equivalents to the quantities.

It should be noted that, as used in this specification and the appended claims, the singular forms "a", "an", and "the" include plural referents unless the content clearly dictates otherwise.

It should be noted that, as used in this specification and the appended claims, "*" if not otherwise defined indicates the chemical bonding position.

The anode electrode and cathode electrode may be described as anode electrode / cathode electrode or anode electrode / cathode electrode or anode electrode layer / cathode electrode layer.

According to another aspect, an organic optoelectronic device comprises an anode layer and a cathode layer facing each other and at least one organic semiconductor layer between the anode layer and the cathode layer, wherein the organic semiconductor layer comprises or consists of the compound of formula 1.

According to yet another aspect, a display device comprising the organic electronic device, which can be an organic optoelectronic device, is provided.

In the present specification, when a definition is not otherwise provided, an "alkyl group" may refer to an aliphatic hydrocarbon group. The alkyl group may refer to "a saturated alkyl group" without any double bond or triple bond. The alkyl group may be a linear, cyclic or branched alkyl group.

The alkyl group may be a $C_1$ to $C_{16}$ alkyl group, or preferably a $C_1$ to $C_{12}$ alkyl group. More specifically, the alkyl group may be a $C_1$ to $C_{14}$ alkyl group, or preferably a $C_1$ to $C_{10}$ alkyl group or a $C_1$ to $C_6$ alkyl group. For example, a $C_1$ to $C_4$ alkyl group comprises 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, and the like.

In the present specification "arylene group" may refer to a group comprising at least one hydrocarbon aromatic moiety, and all the elements of the hydrocarbon aromatic moiety may have p-orbitals which form conjugation, for example a phenyl group, a naphthyl group, an anthracenyl group, a phenanthrenyl group, a pyrenyl group, a fluorenyl group and the like.

The term "heteroarylene" may refer to aromatic heterocycles with at least one heteroatom, and all the elements of the hydrocarbon heteroaromatic moiety may have p-orbitals which form conjugation.

The term "hetero-fluorene ring" refers to a dibenzo[d,d]furanyl, dibenzo[b,d]thiophenyl or dibenzo[b,d]selenophenyl group.

The heteroatom may be selected from N, O, S, B, Si, P, Se, preferably from N, O and S.

A heteroarylene ring may comprise at least 1 to 3 heteroatoms. Preferably a heteroarylene ring may comprise at least 1 to 3 heteroatoms individually selected from N, S and/or O.

Further preferred in addition to the group of formula 1 at least one additional heteroaryl/ene ring may comprise at least 1 to 3 N-atoms, or at least 1 to 2-N atoms or at least one N-atom.

According to another preferred embodiment the compound according to formula 1 may comprise:

- at least 6 to 25 aromatic rings, preferably at least 7 to 22 aromatic rings, further preferred at least 8 to 20 aromatic rings, in addition preferred at least 9 to 15 aromatic rings and more preferred at least 10 to 14 aromatic rings; wherein
- at least 2 to 5, preferably 3 to 4 or 2 to 3 are heteroaromatic rings.

According to one embodiment the compound according to formula 1:

- comprises at least 6 to 20 aromatic rings, preferably at least 7 to 18 aromatic rings, further preferred at least 9 to 16 aromatic rings, in addition preferred at least 10 to 15 aromatic rings and more preferred at least 11 to 14 aromatic rings; and/or
- the compound of formula 1 comprises at least 2 to 6, preferably 3 to 5 or 2 to 4, hetero aromatic rings, wherein the hetero atoms can be selected from N, O, S; and/or
- comprises at least one fluorene ring and at least one hetero-fluorene ring, wherein the hetero atoms can be selected from N, O, S; and/or
- comprises at least one ring, or at least two rings.

According to a further preferred embodiment the compound of formula 1 comprises at least 2 to 7, preferably 2 to 5, or 2 to 3 hetero aromatic rings.

According to a further preferred embodiment the compound of formula 1 comprises at least 2 to 7, preferably 2 to 5, or 2 to 3 hetero aromatic rings, wherein at least one of the aromatic rings is a five member hetero aromatic ring.

According to a further preferred embodiment the compound of formula 1 comprises at least 3 to 7, preferably 3 to 6, or 3 to 5 hetero aromatic rings, wherein at least two of the hetero aromatic rings are five member hetero-aromatic-rings.

According to one embodiment the compound according to formula 1 may comprise at least 6 to 12 non-hetero aromatic rings and 2 to 3 hetero aromatic rings.

According to one preferred embodiment the compound according to formula 1 may comprise at least 7 to 12 non-hetero aromatic rings and 2 to 5 hetero aromatic rings.

According to one preferred embodiment the compound according to formula 1 may comprise at least 7 to 11 non-hetero aromatic rings and 2 to 3 hetero aromatic rings.

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

According to another embodiment the compound of formula 1 may have a melting point of ≥ 250° C and ≤ 380° C, preferably ≥ 260° C and ≤ 370° C, further preferred ≥ 270° C and ≤ 360° C, in addition preferred ≥ 275° C and ≤ 350° C, also preferred ≥ 280° C and ≤ 340° C, likewise preferred ≥ 285° C and ≤ 330° C and more preferred ≥ 290° C and ≤ 310° C.

Glass transition temperature

The glass transition temperature is measured under nitrogen and using a heating rate of 10 K per min in a Mettler Toledo DSC 822e differential scanning calorimeter as described in DIN EN ISO 11357, published in March 2010.

According to another embodiment the compound of formula 1 may have a glass transition temperature Tg of ≥ 120° C and ≤ 380° C, preferably ≥ 130° C and ≤ 350° C, further preferred ≥ 140° C and ≤ 320° C, in addition preferred ≥ 140° C and ≤ 200° C and also preferred ≥ 140° C and ≤ 180° C.

Rate onset temperature

The rate onset temperature is determined by loading 100 mg compound into a VTE source. The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Ångstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low takt time and decomposition of the organic compound in VTE source may occur due to prolonged exposure to elevated temperatures.

The rate onset temperature is an indirect measure of the volatility of a compound. The higher the rate onset temperature the lower is the volatility of a compound.

According to another embodiment the compound of formula 1 may have a rate onset temperature $T_{RO}$ of ≥ 200° C and ≤ 350° C, preferably ≥ 220° C and ≤ 350° C, further preferred ≥ 240° C and ≤ 320° C, in addition preferred ≥ 240° C and ≤ 300° C and more preferred ≥ 240° C and ≤ 280° C.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method. The geometries of the molecular structures are optimized using the hybrid functional B3LYP with the 6-31G* basis set in the gas phase as implemented

in the program package TURBOMOLE V6.5. If more than one conformation is viable, the conformation with the lowest total energy is selected to determine the bond lengths of the molecules.

According to one embodiment the compounds according to formula 1 may have a dipole moment (Debye) in the range from ≥ 0.25 to ≤ 1.50, preferably from ≥ 0.28 to ≤ 1.00, further preferred from ≥ 0.30 to ≤ 0.70, also preferred from ≥ 0.32 to ≤ 0.60.

Calculated HOMO and LUMO

The HOMO and LUMO are calculated with the program package TURBOMOLE V6.5. The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

According to one embodiment the compounds according to formula 1 may have a LUMO energy level (eV) in the range from - 2.10 eV to - 1.90 eV, preferably from - 1.99 eV to - 1.91 eV, further preferred from - 1.98 eV to - 1.92 eV, also preferred from - 1.97 eV to - 1.93 eV.

To determine the distribution of the LUMO in the materials, the isosurfaces of the orbitals are plotted in the graphical user interface TMolex Version 3.3. The 3D data for this property is generated using a coarse grid and a value of 0.03 was chosen to plot the positive and negative iso-densities of the isosurfaces.

Technical effect

Surprisingly, it was found that the compounds of formula 1 and the inventive organic electronic devices solve the problem underlying the present invention by being superior over the organic electroluminescent devices and compounds known in the art, in particular with respect to cd/A efficiency, also referred to as current efficiency and to lifetime. At the same time the operating voltage is kept at a similar or even improved level which is important for reducing power consumption and increasing battery life, for example of a mobile display device. High cd/A efficiency is important for high efficiency and thereby increased battery life of a mobile device, for example a mobile display device. Long lifetime at high current density is important for the longevity of a device which is run at high brightness.

Additionally, it was surprisingly found that the calculated LUMO level of compounds of formula 1 is significantly more negative than the LUMO of the state of the art. A more negative LUMO may be beneficial for improved electron transfer from the cathode to the emission layer.

Additionally, it was found that in compounds of formula 1 the LUMO is delocalized over the atoms in the triazine group and over the majority of atoms in the dibenzo[d,d]furanyl, dibenzo[b,d]thiophenyl or dibenzo[b,d]selenophenyl group and in the triazinyl group. Without being bound by theory, more efficient electron transfer from neighbouring molecules and/or the cathode and/or improved stability of the molecule itself may be achieved when the LUMO is delocalized over the atoms in the triazine group and over the majority of atoms in the dibenzo[d,d]furanyl, dibenzo[b,d]thiophenyl or dibenzo[b,d]selenophenyl group and in the triazinyl group.

Futhermore, it was found that compounds of formula 1 have a high Tg. A high Tg may be beneficial for improved stability of the organic semiconductor layer, in particular at elevated temperatures, for example for application in automobiles.

The inventors have surprisingly found that particular good performance can be achieved when using the organic electroluminescent device as a fluorescent blue device.

The specific arrangements mentioned herein as preferred were found to be particularly advantageous.

Likewise, some compounds falling within the scope of the broadest definition of the present invention have surprisingly be found to be particularly well performing with respect to the mentioned property of cd/A efficiency and/or lifetime. These compounds are discussed herein to be particularly preferred.

Further an organic optoelectronic device having high efficiency and/or long lifetime may be realized.

Anode

A material for the anode may be a metal or a metal oxide, or an organic material, preferably a material with work function above 4.8 eV, more preferably above 5.1 eV, most preferably above 5.3 eV. Preferred metals are noble metals like Pt, Au or Ag, preferred metal oxides are transparent metal oxides like ITO or IZO which may be advantageously used in bottom-emitting OLEDs having a reflective cathode.

In devices comprising a transparent metal oxide anode or a reflective metal anode, the anode may have a thickness from 50 nm to 100 nm, whereas semitransparent metal anodes may be as thin as from 5 nm to 15 nm, and non-transparent metal anodes may have a thickness from 15 nm to 150nm.

Hole injection layer (HIL)

The hole injection layer may improve interface properties between the anode and an organic material used for the hole transport layer, and is applied on a non-planarized anode and thus may planarize the surface of the anode. For example, the hole injection layer may include a material having a median value of the energy level of its highest occupied molecular orbital (HOMO) between the work function of the anode material and the energy level of the HOMO of the hole transport layer, in order to adjust a difference between the work function of the anode and the energy level of the HOMO of the hole transport layer.

When the hole transport region comprises a hole injection layer 36, the hole injection layer may be formed on the anode by any of a variety of methods, for example, vacuum deposition, spin coating, casting, Langmuir-Blodgett (LB) method, or the like.

When hole injection layer is formed using vacuum deposition, vacuum deposition conditions may vary depending on the material that is used to form the hole injection layer, and the desired structure and thermal properties of the hole injection layer to be formed and for example, vacuum deposition may be performed at a temperature of 100 °C to 500 °C, a pressure of $10^{-6}$ Pa to $10^{-1}$ Pa, and a deposition rate of 0.1 to 10 nm/sec, but the deposition conditions are not limited thereto.

When the hole injection layer is formed using spin coating, the coating conditions may vary depending on the material that is used to form the hole injection layer, and the desired structure and thermal properties of the hole injection layer to be formed. For example, the coating rate may be in the range of 2000 rpm to 5000 rpm, and a temperature at which heat treatment is performed to remove a solvent after coating may be in a range of 80 °C to 200 °C, but the coating conditions are not limited thereto.

The hole injection layer may further comprise a p-dopant to improve conductivity and/or hole injection from the anode.

p-dopant

In another aspect, the p-dopant may be homogeneously dispersed in the hole injection layer.

In another aspect, the p-dopant may be present in the hole injection layer in a higher concentration closer to the anode and in a lower concentration closer to the cathode.

The p-dopant may be one of a quinone derivative or a radialene compound but not limited thereto. Non-limiting examples of the p-dopant are quinone derivatives such as tetracyanoquinonedimethane (TCNQ), 2,3,5,6-tetrafluoro-tetracyano-1,4-benzoquinonedimethane (F4-TCNQ), 4,4',4"-((1E,1'E,1"E)-cyclopropane-1,2,3-triylidenetris(cyanomethanylylidene))-tris(2,3,5,6-tetrafluorobenzonitrile).

Acccording to another embodiment, an organic electronic device comrising an organic semiconductor layer that comprises a compound of formula 1 and a dopant selected from an organic metal complex or metal halide may additional comprise a layer comprising a radialene compound and/or a quinodimethane compound.

In another embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perΣluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have formula (XX) and/or the quinodimethane compound may have formula (XXIa) or (XXIb):

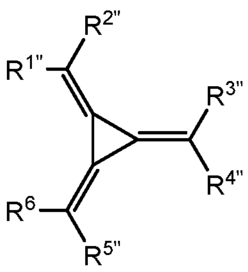 (XX) 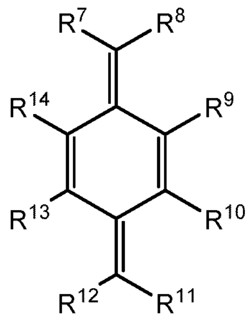 (XXIa)

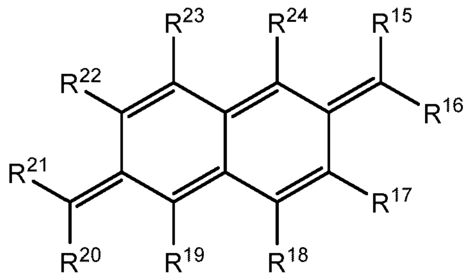 (XXIb),

wherein $R^{1"}$, $R^{2"}$, $R^{3"}$, $R^{4"}$, $R^{5"}$, $R^6$, $R^7$, $R^8$, $R^{11}$, $R^{12}$, $R^{15}$, $R^{16}$, $R^{20}$, $R^{21}$ are independently selected from an electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and electron withdrawing groups. Electron withdrawing group/s that can be suitable used are above mentioned.

Hole transport layer (HTL)

Conditions for forming the hole transport layer and the electron blocking layer may be defined based on the above-described formation conditions for the hole injection layer.

A thickness of the hole transport part of the charge transport region may be from 10 nm to 1000 nm, for example, 10 nm to 100 nm. When the hole transport part of the charge transport region comprises the hole injection layer and the hole transport layer, a thickness of the hole injection layer may be from 10 nm to 1000 nm, for example 10 nm to 100 nm and a thickness of the hole transport layer may be from 5 nm to 200 nm, for example 10 nm to 150 nm. When the thicknesses of the hole transport part of the charge transport region, the HIL, and the HTL are within these ranges, satisfactory hole transport characteristics may be obtained without a substantial increase in operating voltage.

Hole transport matrix materials used in the hole transport region are not particularly limited. Preferred are covalent compounds comprising a conjugated system of at least 6 delocalized electrons, preferably organic compounds comprising at least one aromatic ring, more preferably organic compounds comprising at least two aromatic rings, even more preferably organic compounds comprising at least three aromatic rings, most preferably organic compounds comprising at least four aromatic rings. Typical examples of hole transport matrix materials which are widely used in hole transport layers are polycyclic aromatic hydrocarbons, triarylene amine compounds and heterocyclic aromatic compounds. Suitable ranges of frontier orbital energy levels of hole transport matrices useful in various layer of the hole transport region are well-known. In terms of the redox potential of the redox couple HTL matrix/ cation radical of the HTL matrix, the preferred values (if measured for example by cyclic voltammetry against ferrocene/ferrocenium redox couple as reference) may be in the range 0.0 - 1.0 V, more preferably in the range 0.2 - 0.7 V, even more preferably in the range 0.3 - 0.5 V.

Buffer layer

The hole transport part of the charge transport region may further include a buffer layer.

Buffer layer that can be suitable used are disclosed in US 6 140 763, US 6 614 176 and in US2016/248022.

The buffer layer may compensate for an optical resonance distance of light according to a wavelength of the light emitted from the EML, and thus may increase efficiency.

Emission layer (EML)

The emission layer may be formed on the hole transport region by using vacuum deposition, spin coating, casting, LB method, or the like. When the emission layer is formed using vacuum deposition or spin coating, the conditions for deposition and coating may be similar to those for the formation of the hole injection layer, though the conditions for the deposition and coating may vary depending on the material that is used to form the emission layer. The emission layer may include an emitter host (EML host) and an emitter dopant (further only emitter).

A thickness of the emission layer may be 100Å to 1000Å, for example 200Å to 600Å. When the thickness of the emission layer is within these ranges, the emission layer may have improved emission characteristics without a substantial increase in operating voltage.

Emitter host

According to another embodiment, the emission layer comprises compound of formula 1 as emitter host.

The emitter host compound has at least three aromatic rings, which are independently selected from carbocyclic rings and heterocyclic rings.

Other compounds that can be used as the emitter host is an anthracene matrix compound represented by formula 400 below:

Formula 400

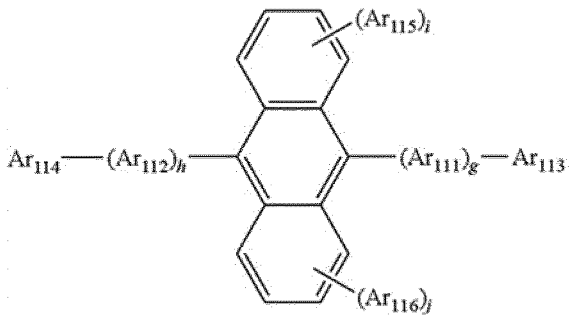

In formula 400, $Ar_{111}$ and $Ar_{112}$ may be each independently a substituted or unsubstituted $C_6$-$C_{60}$ arylene group; $Ar_{113}$ to $Ar_{116}$ may be each independently a substituted or unsubstituted $C_1$-$C_{10}$ alkyl group or a substituted or unsubstituted $C_6$-$C_{60}$ arylene group; and g, h, i, and j may be each independently an integer from 0 to 4.

In some embodiments, Arm and $Ar_{112}$ in formula 400 may be each independently one of a phenylene group, a naphthalene group, a phenanthrenylene group, or a pyrenylene group; or
a phenylene group, a naphthalene group, a phenanthrenylene group, a fluorenyl group, or a pyrenylene group, each substituted with at least one of a phenyl group, a naphthyl group, or an anthryl group.

In formula 400, g, h, i, and j may be each independently an integer of 0, 1, or 2.

In formula 400, $Ar_{113}$ to $Ar_{116}$ may be each independently one of

- a $C_1$-$C_{10}$ alkyl group substituted with at least one of a phenyl group, a naphthyl group, or an anthryl group;
- a phenyl group, a naphthyl group, an anthryl group, a pyrenyl group, a phenanthrenyl group, or a fluorenyl group;
- a phenyl group, a naphthyl group, an anthryl group, a pyrenyl group, a phenanthrenyl group, or a fluorenyl group, each substituted with at least one of a deuterium atom, a halogen atom, a hydroxyl group, a cyano group, a nitro group, an amino group, an amidino group, a hydrazine group, a hydrazone group, a carboxyl group or a salt thereof,
- a sulfonic acid group or a salt thereof, a phosphoric acid group or a salt thereof,
- a $C_1$-$C_{60}$ alkyl group, a $C_2$-$C_{60}$ alkenyl group, a $C_2$-$C_{60}$ alkynyl group, a $C_1$-$C_{60}$ alkoxy group, a phenyl group, a naphthyl group, an anthryl group, a pyrenyl group, a phenanthrenyl group, or
- a fluorenyl group

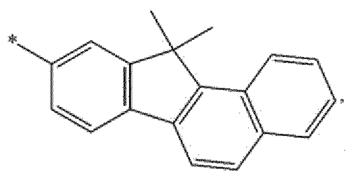

;

or

- formulas 7 or 8

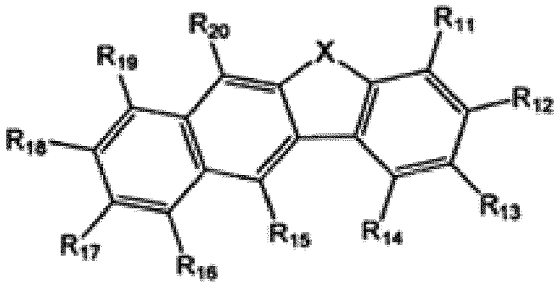

(7),

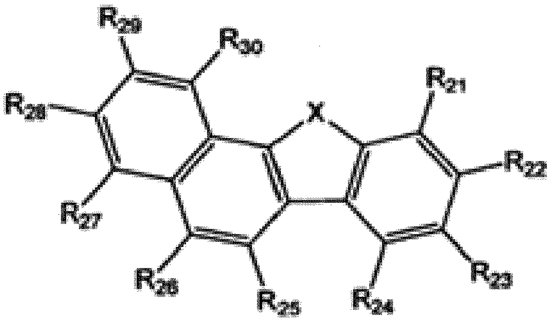

(8).

Wherein in the formulas 7 and 8, X is selected form an oxygen atom and a sulfur atom, but embodiments of the invention are not limited thereto.

In the formula 7, any one of $R_{11}$ to $R_{14}$ is used for bonding to Arm. $R_{11}$ to $R_{14}$ that are not used for bonding to Arm and $R_{15}$ to $R_{20}$ are the same as $R_1$ to $R_8$.

In the formula 8, any one of $R_{21}$ to $R_{24}$ is used for bonding to Arm. $R_{21}$ to $R_{24}$ that are not used for bonding to Arm and $R_{25}$ to $R_{30}$ are the same as $R_1$ to $R_8$.

Preferably, the EML host comprises between one and three heteroatoms selected from the group consisting of N, O or S. More preferred the EML host comprises one heteroatom selected from S or O.

Emitter dopant

The dopant is mixed in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The emitter may be a red, green, or blue emitter.

The dopant may be a fluorescent dopant, for example ter-fluorene, the structures are shown below. 4.4'-bis(4-diphenyl amiostyryl)biphenyl (DPAVBI, 2,5,8,11-tetra-tert-butyl perylene (TBPe), and Compound 8 below are examples of fluorescent blue dopants.

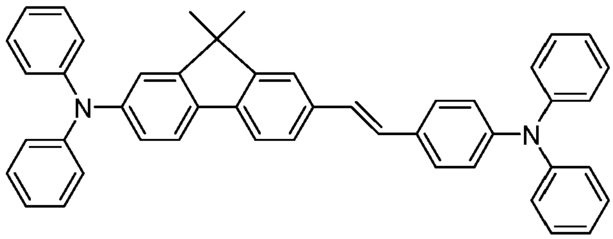

**Compound 8**

The dopant may be a phosphorescent dopant, and examples of the phosphorescent dopant may be an organic metal compound comprising Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto:

$J_2MX$ (Z).

In formula Z, M is a metal, and J and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or a combination thereof, and the J and X may be, for example a bidendate ligand.

One or more emission layers may be arranged between the anode and the cathode. To increase overall

performance, two or more emission layers may be present.

Charge generation layer

A charge generation layer (also named CGL) may be arranged between the first and the second emission layer, and second and third emission layer, if present. Typically, the CGL comprises a n-type charge generation layer (also named n-CGL or electron generation layer) and a p-type charge generation layer (also named p-CGL or hole generation layer). An interlayer may be arranged between the n-type CGL and the p-type CGL.
In one aspect, the n-type CGL may comprise a compound of formula 1. The n-type CGL , further comprises a metal, metal salt or organic metal complex, preferably a metal. The metal may be selected from an alkali, alkaline earth or rare earth metal.
The p-type CGL may comprise a dipyrazino[2,3-f:2',3'-h]quinoxaline, a quinone compound or a radialene compound, preferably dipyrazino[2,3-f:2',3'-h]quinoxaline-2,3,6,7,10,11-hexacarbonitrile or a compound or formula (XX) and/or a compound of formula (XXIa) or (XXIb).

Electron transport layer (ETL)

According to another embodiment, the organic semiconductor layer that comprises compound of formula 1 is an electron transport layer. In another embodiment the electron transport layer may consist of compound of formula 1.
For example, an organic light emitting diode according to an embodiment of the present invention comprises at least one electron transport layer, and in this case, the electron transport layer comprises compound of formula 1, or preferably of at least one compound of formulae D1 to D8.
In another embodiment, the organic electronic device comprises an electron transport region of a stack of organic layers formed by two or more electron transport layers, wherein at least one electron transport layer comprises compound of formula 1 and a dopant selected from an organic metal complex or metal halide.
The electron transport layer may include one or two or more different electron transport compounds.
According to another embodiment, a first and a second electron transport layers comprise compound of formula 1 and a dopant selected from an organic metal complex or metal halide, wherein the compound of formula 1 is not selected the same.
The thickness of the first electron transport layer may be from 0.5 nm to 100 nm, for example 2 nm to 40 nm. When the thickness of the first electron transport layer is within these ranges, the first electron transport layer may have improved electron transport ability without a substantial increase in operating voltage.
A thickness of an optional second electron transport layer may be 1 nm to 100 nm, for example 2 nm to 20 nm. When the thickness of the electron transport layer is within these ranges, the electron transport layer may have satisfactory electron transporting ability without a substantial increase in operating voltage.
The dopant of the electron transport layer may be a monovalent or divalent metal halide or an organic monovalent or divalent metal complex, preferably an alkali halide and/or alkali organic complex.
According to another embodiment, the first and second electron transport layers comprise compound of formula 1, wherein the second electron transport layer further comprises the dopant selected from an organic metal complex or metal halide, preferably an alkali halide and/or alkali organic complex.

Alkali halide

Alkali halides that can be suitable used as the dopant (metal halide), also known as alkali metal halides, are the family of inorganic compounds with the chemical formula MX, where M is an alkali metal and X is a halogen.
M can be selected from Li, Na, Potassium, Rubidium and Cesium.
X can be selected from F, Cl, Br and J.
According to various embodiments of the present invention a lithium halide may be preferred. The lithium halide can be selected from the group comprising LiF, LiCl, LiBr and LiJ. However, most preferred is LiF.
The alkali halide is essentially non-emissive or non-emissive.

Alkali organic complex

The alkali organic complex that can be suitable used as the dopant as organic metal complex comprises an alkali metal and at least one organic ligand. The alkali metal is preferably selected from lithium.
According to various embodiments of the present invention the organic ligand of the lithium organic complex is a quinolate, a borate, a phenolate, a pyridinolate or a Schiff base ligand;

- preferably the lithium quinolate complex has the formula III, IV or V:

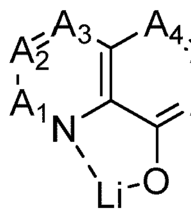 (III), 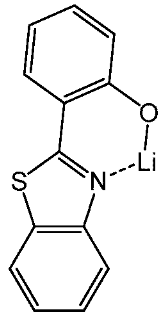 (IV), 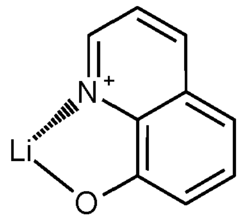 (V),

wherein

$A_1$ to $A_6$ are same or independently selected from CH, CR, N and O;
R is same or independently selected from hydrogen, halogen, alkyl or arylene or heteroarylene with 1 to 20 carbon atoms; and more preferred A1 to A6 are CH;

- preferably the borate based organic ligand is a tetra(1H-pyrazol-1-yl)borate;
- preferably the phenolate is a 2-(pyridin-2-yl)phenolate, a 2-(diphenylphosphoryl)phenolate, an imidazol phenolates, or 2-(pyridin-2-yl)phenolate and more preferred 2-(1-phenyl-1H-benzo[d]imidazol-2-yl)phenolate;
- preferably the pyridinolate is a 2-(diphenylphosphoryl)pyridin-3-olate.

According to various embodiments of the present invention the organic ligand of the alkali organic complex, preferably of a lithium organic complex, can be a quinolate. Quinolates that can be suitable used are disclosed in WO 2013079217 A1 and incorporated by reference.
According to various embodiments of the present invention the organic ligand of the lithium organic complex can be a borate based organic ligand, Preferably the lithium organic complex is a lithium tetra(1H-pyrazol-1-yl)borate. Borate based organic ligands that can be suitable used are disclosed in WO 2013079676 A1 and incorporated by reference.
According to various embodiments of the present invention the organic ligand of the lithium organic complex can be a phenolate ligand, Preferably the lithium organic complex is a lithium 2-(diphenylphosphoryl)phenolate. Phenolate ligands that can be suitable used are disclosed in WO 2013079678 A1 and incorporated by reference.
Further, phenolate ligands can be selected from the group of pyridinolate, preferably 2-(diphenylphosphoryl)pyridin-3-olate. Pyridine phenolate ligands that can be suitable used are disclosed in JP 2008195623 and incorporated by reference.
In addition, phenolate ligands can be selected from the group of imidazol phenolates, preferably 2-(1-phenyl-1H-benzo[d]imidazol-2-yl)phenolate. Imidazol phenolate ligands that can be suitable used are disclosed in JP 2001291593 and incorporated by reference.
Also, phenolate ligands can be selected from the group of oxazol phenolates, preferably 2-(benzo[d]oxazol-2-yl)phenolate. Oxazol phenolate ligands that can be suitable used are disclosed in US 20030165711 and incorporated by reference.
The alkali organic complex may be essentially non-emissive.

Electron injection layer (EIL)

According to another aspect of the invention, the organic electroluminescent device may further comprise an electron injection layer between the electron transport layer (first-ETL) and the cathode.
The electron injection layer (EIL) may facilitate injection of electrons from the cathode.
According to another aspect of the invention, the electron injection layer comprises:

(i) an electropositive metal selected from alkali metals, alkaline earth metals and rare earth metals in substantially elemental form, preferably selected from Li, Na, K, Rb, Cs, Mg, Ca, Sr, Ba, Eu and Yb, more preferably from Li, Na, Mg, Ca, Sr and Yb, even more preferably from Li and Yb, most preferably Yb; and/or
(ii) an alkali metal complex and/or alkali metal salt, preferably the Li complex and/or salt, more preferably a Li quinolinolate, even more preferably a lithium 8-hydroxyquino-linolate, most preferably the alkali metal salt and/or

complex of the second electron transport layer (second-ETL) is identical with the alkali metal salt and/or complex of the injection layer.

The electron injection layer may include at least one selected from LiF, NaCl, CsF, $Li_2O$, and BaO.
A thickness of the EIL may be from 0.1 nm to 10 nm, or 0.3 nm to 9 nm. When the thickness of the electron injection layer is within these ranges, the electron injection layer may have satisfactory electron injection ability without a substantial increase in operating voltage.
The electron injection layer may comprise a compound of formula 1.

Cathode

A material for the cathode may be a metal, an alloy, or an electrically conductive compound that have a low work function, or a combination thereof. Specific examples of the material for the cathode may be lithium (Li), magnesium (Mg), aluminum (Al), aluminum-lithium (Al-Li), calcium (Ca), magnesium-indium (Mg-In), magnesium-silver (Mg-Ag), silver (Ag) etc. In order to manufacture a top-emission light-emitting device having a reflective anode deposited on a substrate, the cathode may be formed as a light-transmissive electrode from, for example, indium tin oxide (ITO), indium zinc oxide (IZO) or silver (Ag).
In devices comprising a transparent metal oxide cathode or a reflective metal cathode, the cathode may have a thickness from 50 nm to 100 nm, whereas semitransparent metal cathodes may be as thin as from 5 nm to 15 nm.

Substrate

A substrate may be further disposed under the anode or on the cathode. The substrate may be a substrate that is used in a general organic light emitting diode and may be a glass substrate or a transparent plastic substrate with strong mechanical strength, thermal stability, transparency, surface smoothness, ease of handling, and water resistance.
Hereinafter, the embodiments are illustrated in more detail with reference to examples. However, the present disclosure is not limited to the following examples.

**Description of the Drawings**

These and/or other aspects and advantages of the present invention will become apparent and more readily appreciated from the following description of the exemplary embodiments, taken in conjunction with the accompanying drawings, of which:

FIG. 1 is a schematic sectional view of an organic light-emitting diode (OLED), according to an exemplary embodiment of the present invention with an emission layer, one electron transport layer and an electron injection layer;
FIG. 2 is a schematic sectional view of an organic light-emitting diode (OLED), according to an exemplary embodiment of the present invention with an emission layer and two electron transport layers;
FIG. 3 is a schematic sectional view of an OLED, according to an exemplary embodiment of the present invention with an emission layer and three electron transport layers;
FIG. 4 is a schematic sectional view of an organic light-emitting diode (OLED), according to an exemplary embodiment of the present invention with an emission layer and one electron transport layer;
FIG. 5 is a schematic sectional view of an organic light-emitting diode (OLED), according to an exemplary embodiment of the present invention with an emission layer and two electron transport layers;
FIG. 6 is a schematic sectional view of an OLED, according to an exemplary embodiment of the present invention with an emission layer and three electron transport layers.

Reference will now be made in detail to the exemplary aspects, examples of which are illustrated in the accompanying drawings, wherein like reference numerals refer to the like elements throughout. The exemplary embodiments are described below, in order to explain the aspects, by referring to the figures.
Herein, when a first element is referred to as being formed or disposed "on" a second element, the first element can be disposed directly on the second element, or one or more other elements may be disposed there between. When a first element is referred to as being formed or disposed "directly on" a second element, no other elements are disposed there between.
The term "contacting sandwiched" refers to an arrangement of three layers whereby the layer in the middle is in direct contact with the two adjacent layers.
The organic light emitting diodes according to an embodiment of the present invention may include a hole transport region; an emission layer; and a first electron transport layer comprising a compound according to formula 1

and a dopant selected from an organic metal complex or metal halide.

FIG. 1 is a schematic sectional view of an organic light-emitting diode 100, according to an exemplary embodiment of the present invention. The OLED 100 comprises an emission layer 150, an electron transport layer (ETL) 161 comprising compound of formula 1 and a dopant selected from an organic metal complex or metal halide, and an electron injection layer 180, whereby the first electron transport layer 161 is disposed directly on the emission layer 150 and the electron injection layer 180 is disposed directly on the first electron transport layer 161.

FIG. 2 is a schematic sectional view of an organic light-emitting diode 100, according to an exemplary embodiment of the present invention. The OLED 100 comprises an emission layer 150 and an electron transport layer stack (ETL) 160 comprising a first electron transport layer 161, and a second electron transport layer 162 comprising a compound of formula 1 and a dopant selected from an organic metal complex or metal halide, whereby the second electron transport layer 162 is disposed directly on the first electron transport layer 161.

FIG. 3 is a schematic sectional view of an organic light-emitting diode 100, according to an exemplary embodiment of the present invention. The OLED 100 comprises an emission layer 150 and an electron transport layer stack (ETL) 160 comprising a first electron transport layer 161, a second electron transport layer 162, and a third electron transport layer 163, whereby the second electron transport layer 162 is disposed directly on the first electron transport layer 161 and the third electron transport layer 163 is disposed directly on the first electron transport layer 162. The first and/or the second and/or the third electron transport layer comprise a compound of formula 1 and a dopant selected from an organic metal complex or metal halide.

FIG. 4 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, one first electron transport layer (ETL) 161, an electron injection layer (EIL) 180, and a cathode electrode 190. The first electron transport layer (ETL) 161 comprises compound of formula 1 and a dopant selected from an organic metal complex or metal halide. The electron transport layer (ETL) 161 is formed directly on the EML 150.

FIG. 5 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a cathode electrode 190. The electron transport layer (ETL) 160 comprises a first electron transport layer 161 and a second electron transport layer 162, wherein the first electron transport layer is arranged near to the anode (120) and the second electron transport layer is arranged near to the cathode (190). The first and/or the second electron transport layer comprise a compound of formula 1 and a dopant selected from an organic metal complex or metal halide.

FIG. 6 is a schematic sectional view of an organic light-emitting diode 100, according to an exemplary embodiment of the present invention. The OLED 100 comprises a substrate 110, a first anode electrode 120, a hole injection layer (HIL) 130, a hole transport layer (HTL) 140, an emission layer (EML) 150, an electron transport layer stack (ETL) 160, an electron injection layer (EIL) 180, and a second cathode electrode 190. The electron transport layer stack (ETL) 160 comprises a first electron transport layer 161, a second electron transport layer 162 and a third electron transport layer 163. The first electron transport layer 161 is formed directly on the emission layer (EML) 150. The first, second and/or third electron transport layer comprise compound of formula 1 that is different for each layer, and a dopant selected from an organic metal complex or metal halide.

Organic semiconductor layer

According to another aspect an organic semiconductor layer may comprise at least one compound of formula 1 and a dopant selected from an organic metal complex or metal halide.

According to one embodiment the organic semiconductor layer may comprises at least one compound of formula 1 and dopant selected from an organic metal complex or metal halide, preferably an alkali metal complex, more preferably LiQ or alkali borate.

According to one embodiment the organic semiconductor layer may comprises at least one compound of formula 1 and a dopant selected from an organic metal complex or metal halide, preferably an organic monovalent or divalent metal complex, more preferably LiQ or alkali borate.

According to one embodiment the organic semiconductor layer may comprises at least one compound of formula 1 and LiQ.

According to one embodiment the organic semiconductor layer may comprises at least one compound of formula 1 and an alkali borate.

According to one embodiment, wherein at least one organic semiconductor layer is arranged between the emission layer and the cathode, preferably between the auxiliary electron transport layer and the cathode.

Preferably, the organic semiconductor layer is contacting sandwiched between the auxiliary electron transport

layer and the cathode layer.

The auxiliary electron transport layer may be free of a dopant. Preferably, it may be free of an emitter dopant or a dopant selected from an organic metal complex or metal halide.

The auxiliary electron transport layer may comprise or consist of an organic compound with a dipole moment ≤ 0 and ≤ 3 Debye, preferably ≤ 0 and ≤ 2 Debye. The organic compound may be selected from a triazine compound or an anthracene compound.

The thickness of the auxiliary electron transport layer may be from 0.5 nm to 100 nm, for example 2 nm to 40 nm. When the thickness of the auxiliary electron transport layer is within these ranges, the first electron transport layer may have improved electron transport ability without a substantial increase in operating voltage.

In another embodiment, the organic semiconductor layer is arranged between the emission layer and the electron transport layer.

According to one embodiment, the organic semiconductor layer is arranged between the first and second emission layer. The organic semiconductor layer can be an electron transport layer, an emission layer, a hole blocking layer, a charge generation layer and/or an electron injection layer, preferably an electron transport layer or a charge generation layer, and more preferred an electron transport layer.

According to one embodiment, the organic semiconductor layer can be arranged between a photoactive layer and a cathode layer, preferably between an emission layer or light-absorbing layer and the cathode layer, preferably the organic semiconductor layer is an electron transport layer.

According to one embodiment, the organic semiconductor layer may comprise at least one alkali halide or alkali organic complex.

An organic semiconductor layer comprises a compound according to formula 1 and a dopant selected from an organic metal complex or metal halide, and is essentially non-emissive or non-emitting.

## Organic electronic device

An organic electronic device according to the invention comprises at least one organic semiconductor layer, wherein at least one organic semiconductor layer comprises a compound according to formula 1 and a dopant selected from an organic metal complex or metal halide.

An organic electronic device according to one embodiment, which comprises at least one organic semiconductor layer that comprises a compound according to formula 1 and a dopant selected from an organic metal complex or metal halide, wherein this layer is essentially non-emissive or non-emitting.

According to one embodiment, the organic electronic device may comprises at least one organic semiconductor layer comprising compound of formula 1 and a dopant selected from an organic metal complex or metal halide that is an electron transport layer, an emission layer, a hole blocking layer, a charge generation layer and/or an electron injection layer, preferably an electron transport layer or a charge generation layer, more preferred an electron transport layer.

An organic electronic device according to one embodiment may include a substrate, an anode layer, an organic semiconductor layer comprising compound of formula 1 and a dopant selected from an organic metal complex or metal halide, and a cathode layer.

The organic electronic device according to according to one embodiment may comprises at least one organic semiconductor layer, wherein the organic semiconductor layer comprising a compound of formula 1 and a dopant selected from an organic metal complex or metal halide is arranged between a photoactive layer and a cathode layer, preferably between an emission layer or light-absorbing layer and the cathode layer, preferably the organic semiconductor layer is an electron transport layer

The organic electronic device according to according to one embodiment may comprises at least one organic semiconductor layer comprising compound of formula 1 and a dopant selected from an organic metal complex or metal halide, wherein the at least one organic semiconductor layer further comprises at least one alkali halide or alkali organic complex.

An organic electronic device according to one embodiment comprises at least one organic semiconductor layer comprising at least one compound of formula 1 and a dopant selected from an organic metal complex or metal halide, at least one anode layer, at least one cathode layer and at least one emission layer, wherein the organic semiconductor layer comprising at least one compound of formula 1 and a dopant selected from an organic metal complex or metal halide is preferably arranged between the emission layer and the cathode layer.

An organic electronic device according to one embodiment comprises at least one organic semiconductor layer comprising at least one compound of formula 1 and a dopant selected from an organic metal complex or metal halide and further comprises at least one alkali halide or alkali organic complex.

An organic electronic device according to one embodiment comprises at least one organic semiconductor layer, at least one anode layer, at least one cathode layer and at least one emission layer, wherein the organic semiconductor layer comprising at least one compound of formula 1 and a dopant selected from an organic metal complex or metal

halide is preferably arranged between the emission layer and the cathode layer. Preferably the at least one organic semiconductor layer is an electron transport layer.

An organic light-emitting diode (OLED) according to the invention may include an anode, a hole transport layer (HTL), an emission layer (EML), an electron transport layer (ETL) comprising at least one compound of formula 1 and a dopant selected from an organic metal complex or metal halide, and a cathode, which are sequentially stacked on a substrate. In this regard, the HTL, the EML, and the ETL are thin films formed from organic compounds.

An organic electronic device according to one embodiment can be a light emitting device, thin film transistor, a battery, a display device or a photovoltaic cell, and preferably a light emitting device. A light emitting device can be an OLED.

According to one embodiment the OLED may have the following layer structure, wherein the layers having the following order:
an anode layer, a hole injection layer, optional a first hole transport layer, optional a second hole transport layer, an emission layer, an electron transport layer comprising compound of formula 1 and a dopant selected from an organic metal complex or metal halide according to the invention, an electron injection layer, and a cathode layer.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of formula 1 according to the invention, and
- a second deposition source to release and a dopant selected from an organic metal complex or metal halide, for example an alkali halide or alkali organic complex, preferably a lithium halide or lithium organic complex;

the method comprising the steps of forming the electron transport layer stack; whereby for an organic light-emitting diode (OLED):

- the first electron transport layer is formed by releasing the compound of formula 1 and a dopant selected from an organic metal complex or metal halide according to the invention from the first deposition source and the dopant of an organic metal complex or metal halide, preferably a lithium halide or lithium organic complex from the second deposition source.

According to various embodiments of the present invention, the method may further include forming on the anode electrode an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, preferably a first electron transport layer is formed on the emission layer and a second electron transport layer is formed on the first electron transport layer and the second electron transport layer comprises a compound of formula 1 and a dopant selected from an organic metal complex or metal halide,
- and finally a cathode electrode is formed,
- optional a hole injection layer, a hole transport layer, and a hole blocking layer, formed in that order between the first anode electrode and the emission layer,
- optional an electron injection layer is formed between the electron transport layer stack and the cathode electrode.

According to various embodiments of the present invention, the method may further include forming an electron injection layer on a first electron transport layer. However, according to various embodiments of the OLED of the present invention, the OLED may not comprise an electron injection layer.

According to various embodiments, the OLED may have the following layer structure, wherein the layers having the following order:

an anode, first hole transport layer, second hole transport layer, emission layer, optional second electron transport layer, first electron transport layer comprising compound of formula 1 and a dopant selected from an organic metal complex or metal halide according to the invention, optional a second electron transport layer, optional an electron injection layer, and a cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

Preparation of compounds of formula 1

Compounds of formula 1 may be prepared as described in KR101537500, KR101537499 and KR 2017-0140115.

The chemical structure, calculated HOMO, LUMO and dipole moment of compounds of formula 1 and comparative example ETM-1 are shown in Table 1.

Table 1

| Referred to as: | Structure | Calculated HOMO (eV) | Calculated LUMO (eV) | Dipole moment (Debye) |
|---|---|---|---|---|
| ETM-1 | 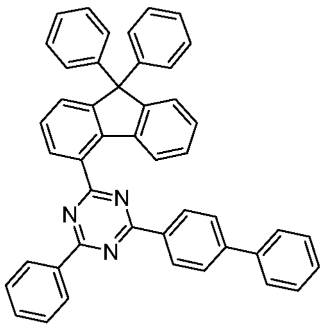 | -5.81 | -1.89 | 0.64 |
| MX1 | 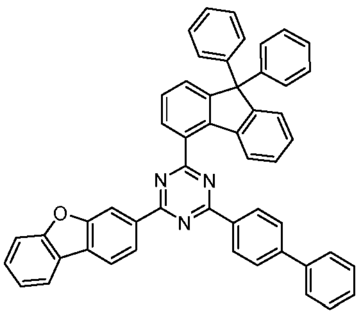 | -5.81 | -1.97 | 0.32 |

(continued)

| Referred to as: | Structure | Calculated HOMO (eV) | Calculated LUMO (eV) | Dipole moment (Debye) |
|---|---|---|---|---|
| MX2 | 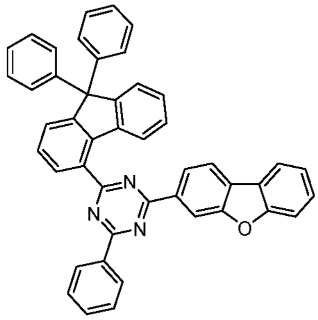 | -5.81 | -1.97 | 0.61 |
| MX3 | 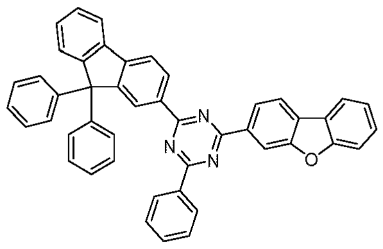 | -5.85 | -1.93 | 0.50 |
| MX4 | 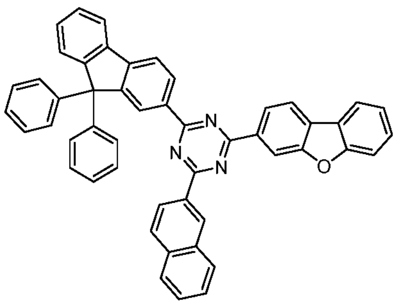 | -5.84 | -1.97 | 0.60 |

As can be seen in Table 1, the LUMO energy level (eV) of compounds of formula 1 are further away from vacuum level (more negative).

General procedure for fabrication of OLEDs

For top emission devices, Examples 1 to 4 and comparative example 1, a glass substrate was cut to a size of 50 mm x 50 mm x 0.7 mm, ultrasonically cleaned with isopropyl alcohol for 5 minutes and then with pure water for 5 minutes, and cleaned again with UV ozone for 30 minutes, to prepare a first electrode. 100 nm Ag were deposited as anode at a pressure of $10^{-5}$ to $10^{-7}$ mbar to form the anode.

Then, 92 vol.-% Biphenyl-4-yl(9,9-diphenyl-9H-fluoren-2-yl)-[4-(9-phenyl-9H-carbazol-3-yl)phenyl]-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the ITO electrode, to form a HIL having a thickness of 10 nm.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a HTL having a thickness of 118 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals) as EML host and 3 vol.-% BD200 (Sun Fine Chemicals) as fluorescent blue dopant were deposited on the EBL, to form a blue-emitting EML with a thickness of 20 nm.

Then the hole blocking layer is formed with a thickness of 5 nm by depositing 2,4-diphenyl-6-(4',5',6'-triphenyl-[1,1':2',1":3",1‴:3‴,1⁗-quinquephenyl]-3⁗-yl)-1,3,5-triazine on the emission layer.

Then, the electron transporting layer is formed on the hole blocking layer according to Examples 1 to 4 and comparative example 1 with a the thickness of 31 nm. The electron transport layer comprises 50 wt.-% matrix compound

and 50 wt.-% of alkali organic complex, see Table 2.

Then, the electron injection layer is formed on the electron transporting layer by deposing Yb with a thickness of 2 nm.

Ag is evaporated at a rate of 0.01 to 1 Å/s at $10^{-7}$ mbar to form a cathode with a thickness of 11 nm.

A cap layer of Biphenyl-4-yl(9,9-diphenyl-9H-fluoren-2-yl)-[4-(9-phenyl-9H-carbazol-3-yl)phenyl]-amine is formed on the cathode with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m$^2$ using an Instrument Systems CAS-140CT array spectrometer (calibrated by Deutsche Akkreditierungsstelle (DAkkS)) for each of the voltage values. The cd/A efficiency at 10 mA/cm$^2$ is determined by interpolating the luminance-voltage and current-voltage characteristics, respectively.

Lifetime LT of the device is measured at ambient conditions (20°C) and 30 mA/cm$^2$, using a Keithley 2400 sourcemeter, and recorded in hours.

The brightness of the device is measured using a calibrated photo diode. The lifetime LT is defined as the time till the brightness of the device is reduced to 97 % of its initial value.

<u>Top emission devices</u>

In Table 2 is shown the performance of organic electronic devices comprising an organic semiconductor layer comprising compound of formula 1 and an alkali organic complex.

In comparative example 1, compound ETM-1 was used as matrix compound:

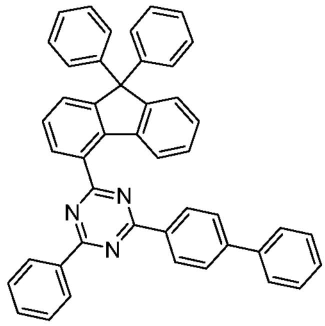

ETM-1.

In comparative example 1, compound ETM-1 was used as matrix compound. The organic semiconductor layer comprises 50 vol.-% ETM-1 and 50 vol.-% LiQ. The operating voltage is 3.4 V and the cd/A efficiency is 7.7 cd/A. The lifetime LT97 at 30 mA/cm2 is 32 hours.

In Example 1, the organic semiconductor layer comprises 50 vol.-% compound of formula 1 MX1 and 50 vol.-% LiQ. The operating voltage is 3.5 V. The cd/A efficiency is 7.7 cd/A and the lifetime is improved to 69 hours.

In Example 2, the organic semiconductor layer comprises 50 vol.-% compound of formula 1 MX2 and 50 vol.-% LiQ. The operating voltage is 3.5 V. The cd/A efficiency is improved to 8 cd/A and the lifetime is improved to 60 hours.

In Example 3, the organic semiconductor layer comprises 50 vol.-% compound of formula 1 MX3 and 50 vol.-% LiQ. The operating voltage is 3.5 V. The cd/A efficiency is 7.7 cd/A and the lifetime is improved to 55 hours.

In Example 4, the organic semiconductor layer comprises 50 vol.-% compound of formula 1 MX4 and 50 vol.-% LiQ. The operating voltage is 3.6 V. The cd/A efficiency is 7.5 cd/A and the lifetime is improved to 51 hours.

Table 2: Performance data of organic electroluminescent device comprising an organic semiconductor layer comprising compound of formula 1 and an alkali organic complex

| | Matrix compound | Concentration of matrix compound (vol.-%) | Alkali organic complex | Concentration of alkali organic complex (vol.-%) | Thickness electron transport layer (nm) | Operating voltage at 10 mA/cm$^2$ (V) | cd/A efficiency at 10 mA/cm$^2$ (cd/A) | LT97 at 30 mA/cm$^2$ (h) |
|---|---|---|---|---|---|---|---|---|
| Comparative example 1 | ETM-1 | 50 | LiQ | 50 | 31 | 3.4 | 7.7 | 32 |
| Example 1 | MX1 | 50 | LiQ | 50 | 31 | 3.5 | 7.5 | 69 |
| Example 2 | MX2 | 50 | LiQ | 50 | 31 | 3.5 | 8.0 | 60 |
| Example 3 | MX3 | 50 | LiQ | 50 | 31 | 3.5 | 7.7 | 55 |
| Example 4 | MX4 | 50 | LiQ | 50 | 31 | 3.6 | 7.5 | 51 |

In summary, improved lifetime and low LUMO energy level (eV) may be achieved when the organic semiconductor layer comprises a compound of formula 1 and a dopant selected from an organic metal complex or metal halide.

While this invention has been described in connection with what is presently considered to be practical exemplary embodiments, it is to be understood that the invention is not limited to the disclosed embodiments, but, on the contrary, is intended to cover various modifications. Therefore, the aforementioned embodiments should be understood to be exemplary but not limiting the present invention in any way.

## Claims

1. An organic semiconductor layer comprising:

   - a compound of formula 1:

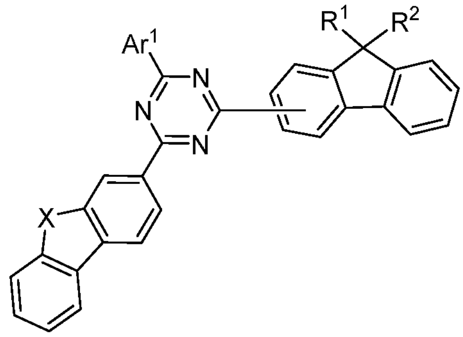

(1),

wherein

X is O, S or Se;

$Ar^1$ is selected from unsubstituted $C_6$ to $C_{24}$ aryl or unsubstituted $C_3$ to C36 heteroaryl ;

$R^1$, $R^2$ are independently selected from substituted or unsubstituted $C_6$ to $C_{24}$ aryl, substituted or unsubstituted $C_3$ to $C_{24}$ heteroaryl, or $R^1$ and $R^2$ are selected from substituted or unsubstituted $C_6$ to $C_{24}$ aryl and substituted or unsubstituted $C_3$ to $C_{24}$ heteroaryl and linked with each other via a single bond such that a five-membered ring is formed,
wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl;

and
   - a dopant selected from an organic metal complex or metal halide.

2. The organic semiconductor layer according to claim 1, wherein the dopant is non-emissive.

3. The organic semiconductor layer according to claim 1 or 2, wherein the dopant is selected from an organic mono- or divalent metal complex, preferably an alkali complex and/or an alkali halide, preferably the dopant is selected from LiQ or lithium borate.

4. The organic semiconductor layer according to any of claims 1 to 3, wherein X is selected from O or S, preferably O.

5. The organic semiconductor layer according to any of claims 1 to 4, wherein

   $R^1$, $R^2$ are independently selected from substituted or unsubstituted $C_6$ to $C_{24}$ aryl, substituted or unsubstituted $C_3$ to $C_{24}$ heteroaryl,

wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl.

**6.** The organic semiconductor layer according to any of claims 1 to 5, wherein

$R^1$, $R^2$ are independently selected from unsubstituted $C_6$ to $C_{24}$ aryl, or unsubstituted $C_3$ to $C_{24}$ heteroaryl.

**7.** The organic semiconductor layer according to any of claims 1 to 6, wherein

$Ar^1$ is selected from unsubstituted $C_6$ to $C_{12}$ aryl, or unsubstituted $C_3$ to $C_{18}$ heteroaryl, preferably phenyl or biphenyl and in addition preferred phenyl.

**8.** The organic semiconductor layer according to any of claims 1 to 7, wherein $Ar^1$ is independently selected from B1 to B 65 and B71 to B77:
wherein

a) B1 to B6 are unsubstituted non-heteroaryl groups:

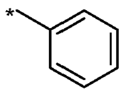
(B1),
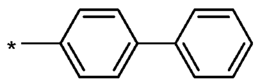
(B2),
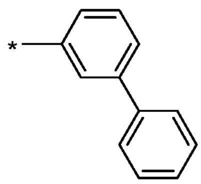
(B3),
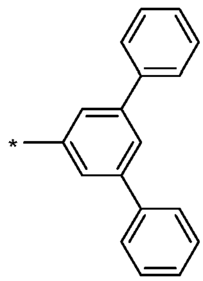
(B4),

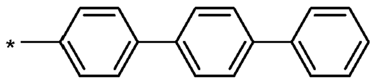
(B5),
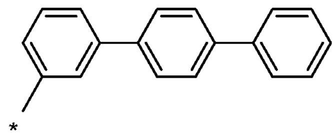
(B6);

or
b) B7 to B23 are unsubstituted annelated non-heteroaryl groups:

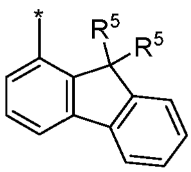
(B7),
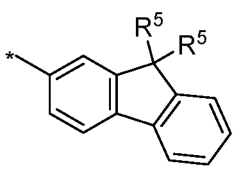
(B8),
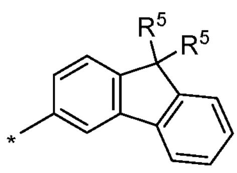
(B9),
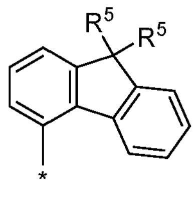
(B10),
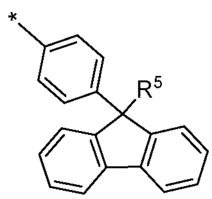
(B11),

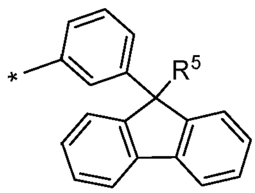
(B12),
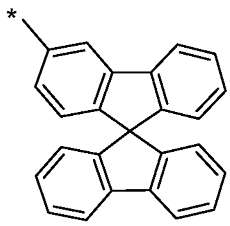
(B13),
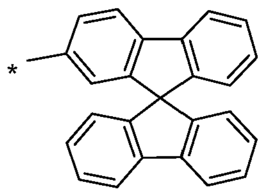
(B14),
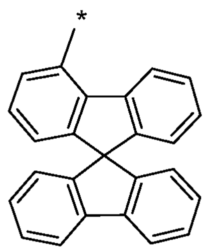
(B15),

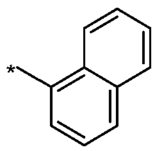
(B16),
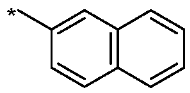
(B17),
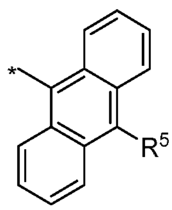
(B18),
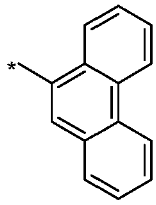
(B19),
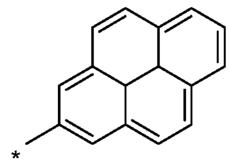
(B20),

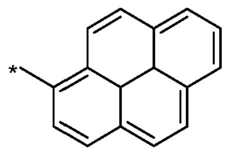
(B21),
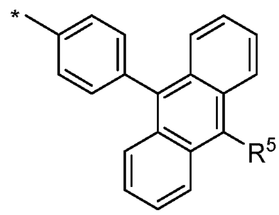
(B22),
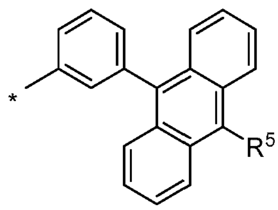
(B23);

or

c) B24 to B31 are dibenzofurane/dibenzothiophene group:

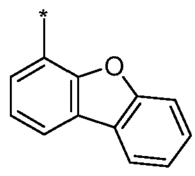
(B24),
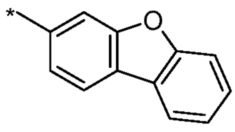
(B25),
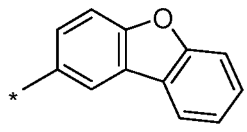
(B26),
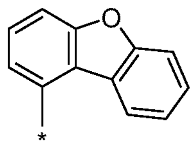
(B27),

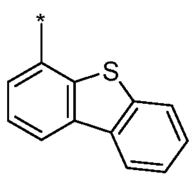
(B28),
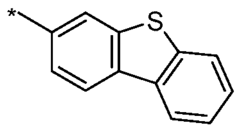
(B29),
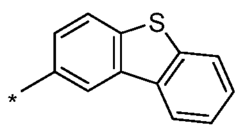
(B30),
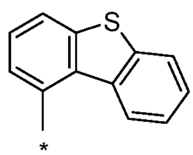
(B31);

or

d) B32 to B34 are unsubstituted pyridine groups:

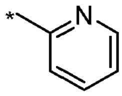 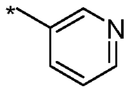 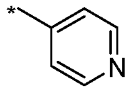

(B32), (B33), (B34);

or

e) B35 to B62 are unsubstituted hetero arylene groups:

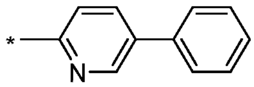 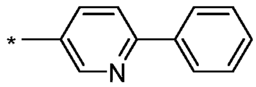

(B35), (B36),

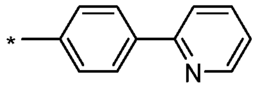 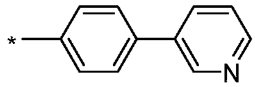 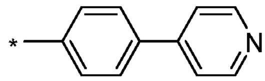

(B37), (B38), (B39),

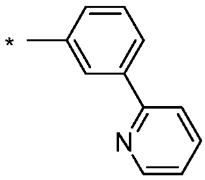 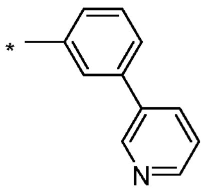 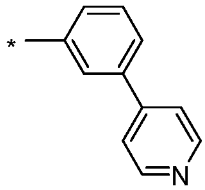 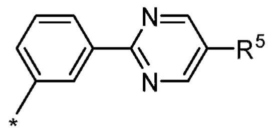

(B40), (B41), (B42), (B43),

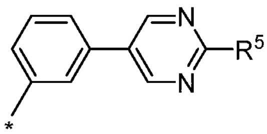 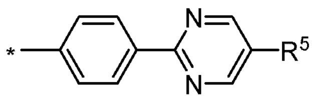 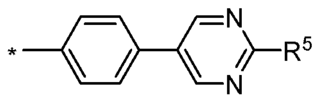

(B44), (B45), (B46),

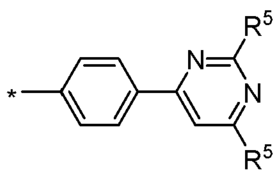 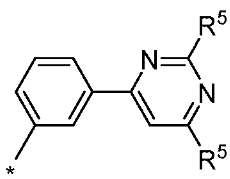 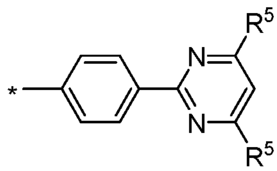

(B47), (B48), (B49),

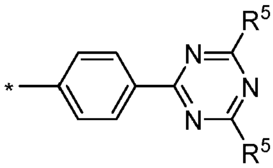
(B50),
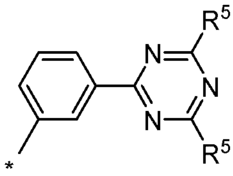
(B51),
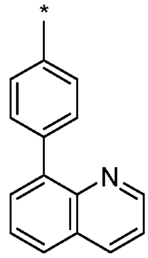
(B52),

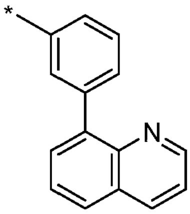
(B53),
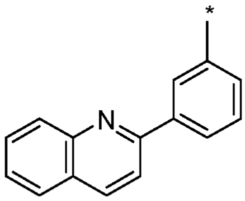
(B54),
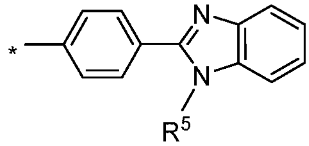
(B55),

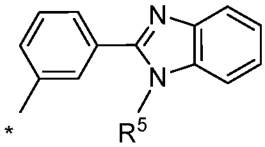
(B56),
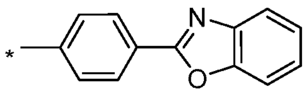
(B57),
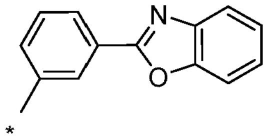
(B58),

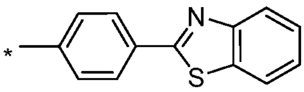
(B59),
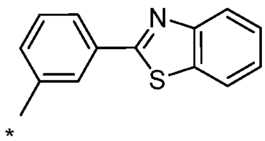
(B60),
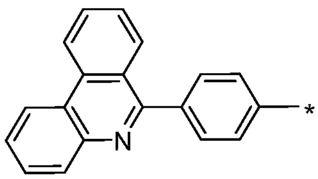
(B61),

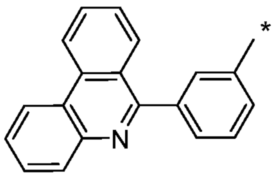
(B62);

or

f) B63 to B65 unsubstituted annelated hetero arylene groups:

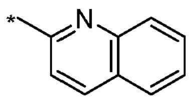 (B63), 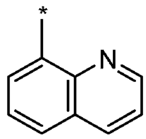 (B64) 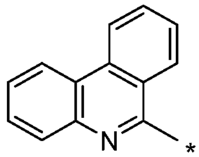 (B65);

or

i) B71 to B77 are carbazole groups

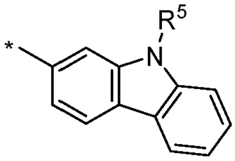 (B71), 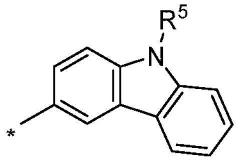 (B72), 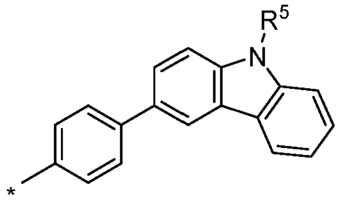 (B73),

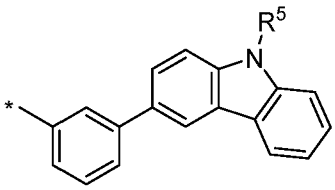 (B74), 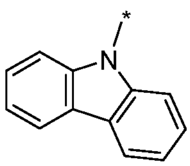 (B75), 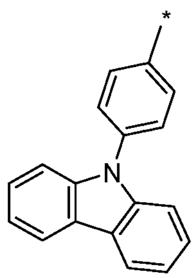 (B76), 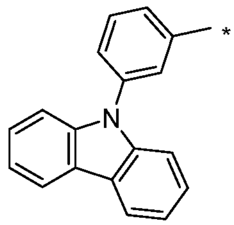 (B77);

wherein

the substituent $R^5$ is H.

**9.** The organic semiconductor layer according to any of claims 1 to 8, wherein the compound of formula 1 is selected from D1 to D8:

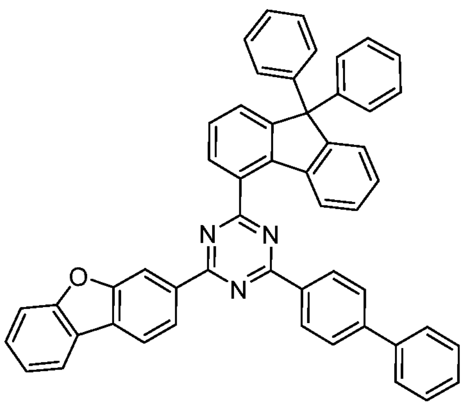 (D1), 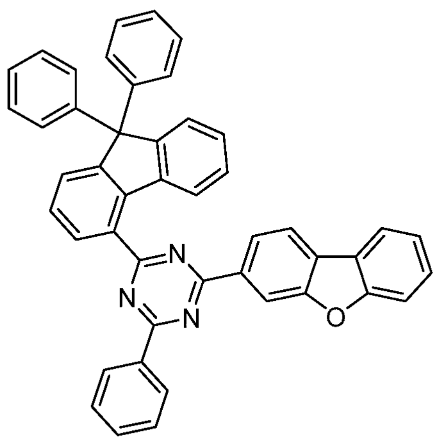 (D2),

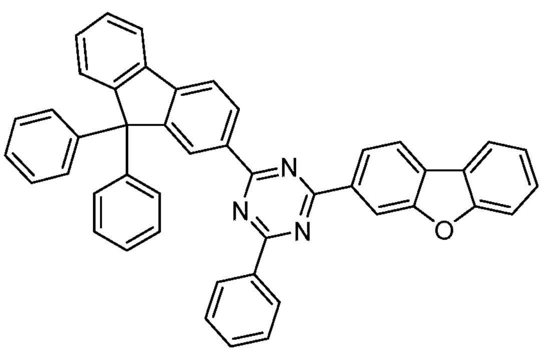

(D3),

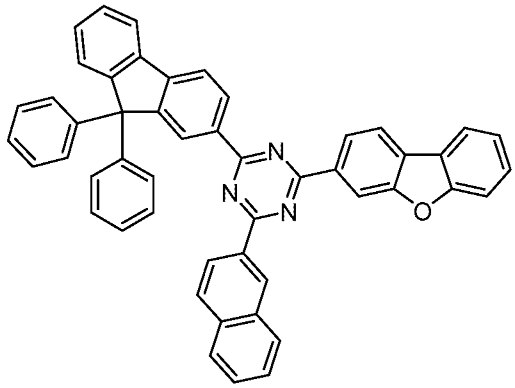

(D4),

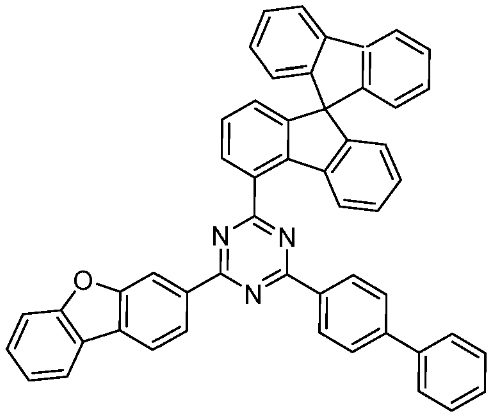

(D5),

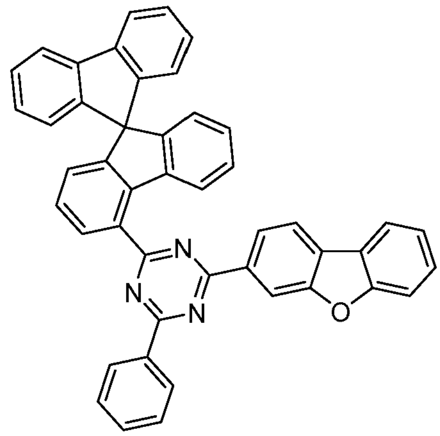

(D6),

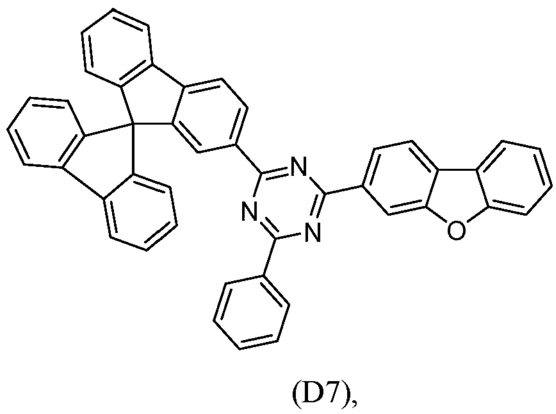

(D7),

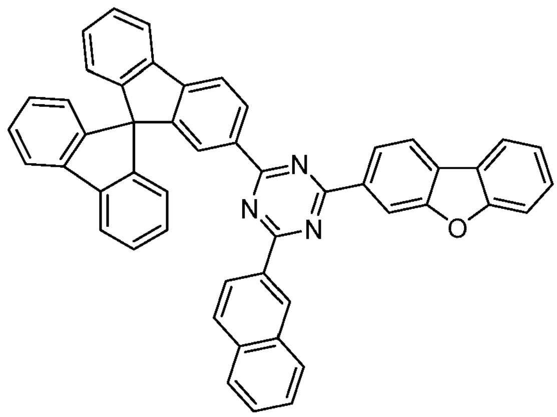

(D8).

**10.** An organic semiconductor layer comprising a compound of formula 1:

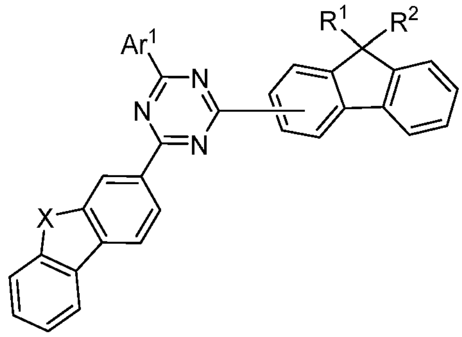

(1),

wherein

X is O, S or Se;

$Ar^1$ is selected from $C_1$ to $C_{16}$ alkyl, substituted or unsubstituted $C_6$ to $C_{40}$ aryl, substituted or unsubstituted $C_3$ to $C_{40}$ heteroaryl, wherein

the substituents of the substituted $C_6$ to $C_{40}$ aryl and substituted $C_3$ to $C_{40}$ heteroaryl are selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, partially or perdeuterated $C_1$ to $C_{16}$ alkoxy, $C_6$ to $C_{24}$ aryl, $C_3$ to $C_{25}$ heteroaryl, $-PXR^3R^4$, D, F or CN, wherein

$R^3$, $R^4$ are independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl, wherein the substituted $C_6$ to $C_{40}$ aryl and/or substituted $C_3$ to $C_{40}$ heteroaryl of $Ar^1$ comprises at least one - $PXR^3R^4$ substituent;

$R^1$, $R^2$ are independently selected from substituted or unsubstituted $C_6$ to $C_{24}$ aryl, substituted or unsubstituted $C_3$ to $C_{24}$ heteroaryl, or optional $R^1$ and $R^2$ are selected from substituted or unsubstituted $C_6$ to $C_{24}$ aryl and substituted or unsubstituted $C_3$ to $C_{24}$ heteroaryl and linked with each other via a single bond such that a five-membered ring is formed, wherein the substituents of the substituted $C_6$ to $C_{24}$ aryl and substituted $C_3$ to $C_{24}$ heteroarylene are selected from H, D, $C_1$ to $C_{16}$ alkyl, partially or perfluorinated $C_1$ to $C_{16}$ alkyl, partially or perdeuterated $C_1$ to $C_{16}$ alkyl, $C_1$ to $C_{16}$ alkoxy, $C_1$ to $C_{16}$ thioalkyl, $C_3$ to $C_{16}$ branched alkyl, $C_3$ to $C_{16}$ cyclic alkyl, $C_3$ to $C_{16}$ branched alkoxy, $C_3$ to $C_{16}$ cyclic alkoxy, $C_3$ to $C_{16}$ branched thioalkyl, $C_3$ to $C_{16}$ cyclic thioalkyl, $C_6$ to $C_{24}$ aryl and $C_3$ to $C_{25}$ heteroaryl;

and

- a dopant selected from an organic metal complex or metal halide.

**11.** The organic semiconductor layer according to claim 10, wherein $Ar^1$ is independently selected from structures C1 to C5:

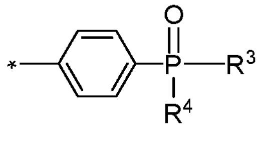

(C1),

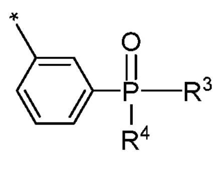

(C2),

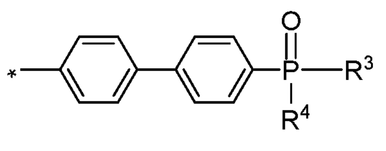

(C3),

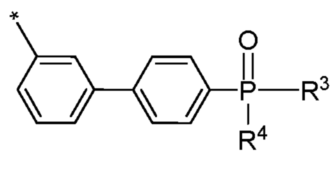

(C4),

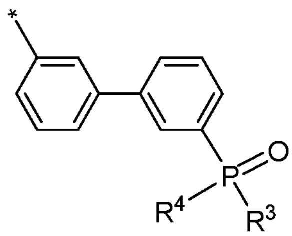

(C5),

wherein $R^3$, $R^4$ is independently selected from $C_1$ to $C_{16}$ alkyl, $C_1$ to $C_{16}$ alkoxy, partially or perfluorinated $C_1$ to $C_{16}$ alkyl, partially or perfluorinated $C_1$ to $C_{16}$ alkoxy, partially or perdeuterated $C_1$ to $C_{16}$ alkyl, partially or perdeuterated $C_1$ to $C_{16}$ alkoxy, $C_6$ to $C_{18}$ aryl, $C_3$ to $C_{25}$ heteroaryl, preferably $R^3$, $R^4$ is selected the same.

**12.** An organic electronic device comprising an organic semiconductor layer according to any of the preceding claims 1 to 11.

**13.** The organic electronic device according to claim 12, wherein the organic semiconductor layer is arranged between an emission layer and a cathode layer, preferably between an auxiliary electron transport layer and a cathode layer, more preferred between an auxiliary electron transport layer and an electron injection layer.

**14.** The organic electronic device according to claim 12 or 13, wherein the electronic device is a light emitting device, thin film transistor, a battery, a display device or a photovoltaic cell, and preferably a light emitting device.

**Patentansprüche**

**1.** Organische Halbleiterschicht, umfassend:

- eine Verbindung der Formel 1:

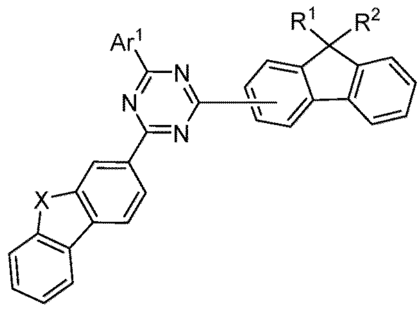

(1),

wobei

X für O, S oder Se steht;
$Ar^1$ aus unsubstituiertem $C_6$- bis $C_{24}$-Aryl oder unsubstituiertem $C_3$- bis $C_{36}$-Heteroaryl ausgewählt ist;
$R^1$, $R^2$ unabhängig aus substituiertem oder unsubstituiertem $C_6$- bis $C_{24}$-Aryl, substituiertem oder unsubstituiertem $C_3$- bis $C_{24}$-Heteroaryl ausgewählt sind oder $R^1$ und $R^2$ aus substituiertem oder unsubstituiertem $C_6$-bis $C_{24}$-Aryl und substituiertem oder unsubstituiertem $C_3$- bis $C_{24}$-Heteroaryl ausgewählt sind und über eine Einfachbindung so miteinander verknüpft sind, dass sich ein fünfgliedriger Ring ergibt,
wobei die Substituenten des substituierten $C_6$- bis $C_{24}$-Aryls und substituierten $C_3$- bis $C_{24}$-Heteroaryls aus H, D, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$-bis $C_{16}$-Alkoxy, $C_1$- bis $C_{16}$-Thioalkyl, verzweigtem $C_3$- bis $C_{16}$-Alkyl, cyclischem $C_3$- bis $C_{16}$-Alkyl, verzweigtem $C_3$- bis $C_{16}$-Alkoxy, cyclischem $C_3$- bis $C_{16}$-Alkoxy, verzweigtem $C_3$- bis $C_{16}$-Thioalkyl, cyclischem $C_3$- bis $C_{16}$-Thioalkyl, $C_6$- bis $C_{24}$-Aryl und $C_3$- bis $C_{25}$-Heteroaryl ausgewählt sind;

und

- einen Dotanden, der aus einem organischen Metallkomplex oder einem Metallhalogenid ausgewählt ist.

**2.** Organische Halbleiterschicht nach Anspruch 1, wobei der Dotand nichtemissiv ist.

**3.** Organische Halbleiterschicht nach Anspruch 1 oder 2, wobei der Dotand aus einem organischen einwertigen oder zweiwertigen Metallkomplex, vorzugsweise einem Alkalikomplex und/oder einem Alkalihalogenid, ausgewählt ist, vorzugsweise der Dotand aus LiQ oder Lithiumborat ausgewählt ist.

**4.** Organische Halbleiterschicht nach einem der Ansprüche 1 bis 3, wobei X aus O oder S, vorzugsweise O, ausgewählt ist.

**5.** Organische Halbleiterschicht nach einem der Ansprüche 1 bis 4, wobei

$R^1$, $R^2$ unabhängig aus substituiertem oder unsubstituiertem $C_6$- bis $C_{24}$-Aryl, substituiertem oder unsubstituiertem $C_3$- bis $C_{24}$-Heteroaryl ausgewählt sind,
wobei die Substituenten des substituierten $C_6$- bis $C_{24}$-Aryls und substituierten $C_3$- bis $C_{24}$-Heteroarylens aus H, D, $C_1$- bis $C_{16}$-Alkyl, teil- oder perfluoriertem $C_1$- bis $C_{16}$-Alkyl, teil- oder perdeuteriertem $C_1$- bis $C_{16}$-Alkyl, $C_1$-bis $C_{16}$-Alkoxy, $C_1$- bis $C_{16}$-Thioalkyl, verzweigtem $C_3$- bis $C_{16}$-Alkyl, cyclischem $C_3$- bis $C_{16}$-Alkyl, verzweigtem $C_3$- bis $C_{16}$-Alkoxy, cyclischem $C_3$- bis $C_{16}$-Alkoxy, verzweigtem $C_3$- bis $C_{16}$-Thioalkyl, cyclischem $C_3$- bis $C_{16}$-Thioalkyl, $C_6$- bis $C_{24}$-Aryl und $C_3$- bis $C_{25}$-Heteroaryl ausgewählt sind.

**6.** Organische Halbleiterschicht nach einem der Ansprüche 1 bis 5, wobei

$R^1$, $R^2$ unabhängig aus unsubstituiertem $C_6$- bis $C_{24}$-Aryl oder unsubstituiertem $C_3$- bis $C_{24}$-Heteroaryl ausgewählt sind.

**7.** Organische Halbleiterschicht nach einem der Ansprüche 1 bis 6, wobei

$Ar^1$ aus unsubstituiertem $C_6$- bis $C_{12}$-Aryl oder unsubstituiertem $C_3$- bis $C_{18}$-Heteroaryl, vorzugsweise Phenyl oder Biphenyl und zusätzlich bevorzugt Phenyl ausgewählt ist.

**8.** Organische Halbleiterschicht nach einem der Ansprüche 1 bis 7, wobei $Ar^1$ unabhängig aus B1 bis B65 und B71 bis B 77 ausgewählt ist:
wobei

a) B1 bis B6 unsubstituierte Nichtheteroarylgruppen sind:

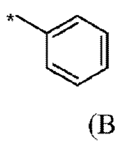
(B1),
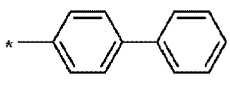
(B2),
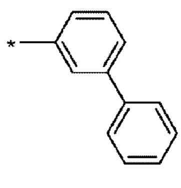
(B3),
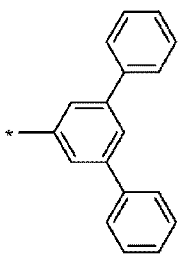
(B4),

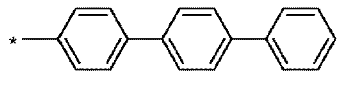
(B5),
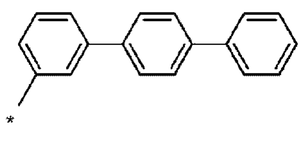
(B6);

oder

b) B7 bis B23 unsubstituierte anellierte Nichtheteroarylgruppen sind:

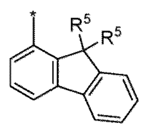 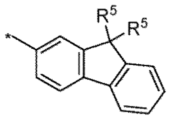 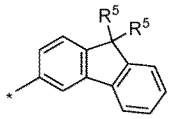 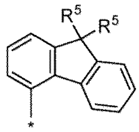 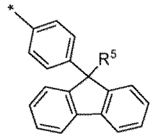

(B7), (B8), (B9), (B10), (B11),

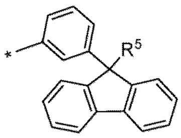 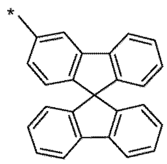 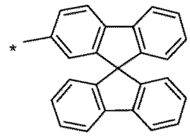 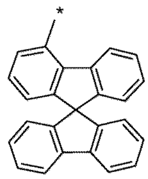

(B12), (B13), (B14), (B15),

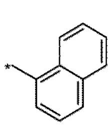 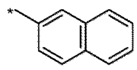 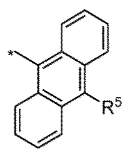 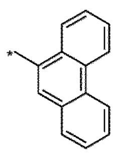 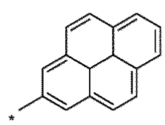

(B16), (B17), (B18), (B19), (B20),

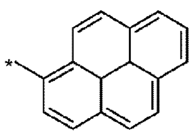 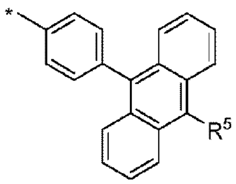 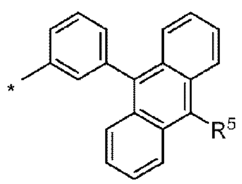

(B21), (B22), (B23);

oder

c) B24 bis B31 Dibenzofuran/Dibenzothiophen-Gruppen sind:

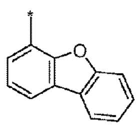 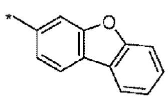 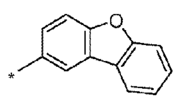 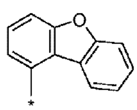

(B24), (B25), (B26), (B27),

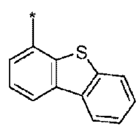 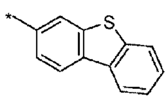 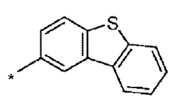 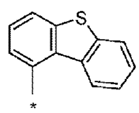

(B28), (B29), (B30), (B31);

oder

d) B32 bis B34 unsubstituierte Pyridingruppen sind:

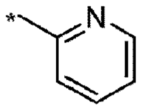 (B32), 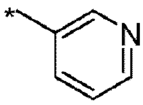 (B33), 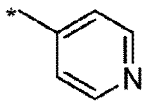 (B34);

oder

e) B35 bis B62 unsubstituierte Heteroarylengruppen sind:

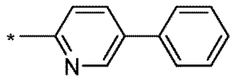 (B35), 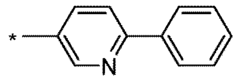 (B36),

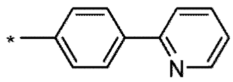 (B37), 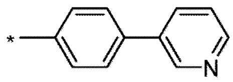 (B38), 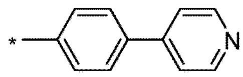 (B39),

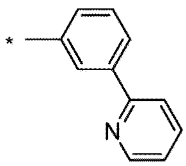 (B40), 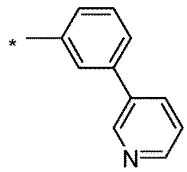 (B41), 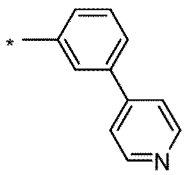 (B42), 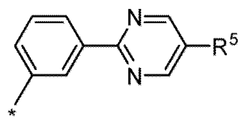 (B43),

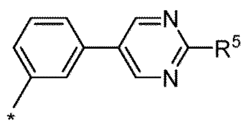 (B44), 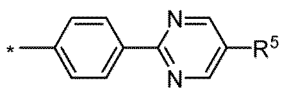 (B45), 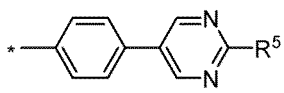 (B46),

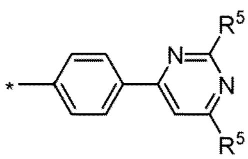 (B47), 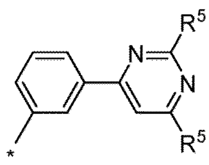 (B48), 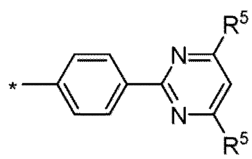 (B49),

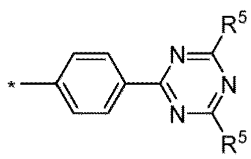 (B50), 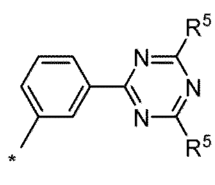 (B51), 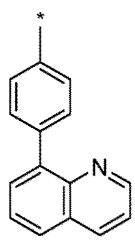 (B52),

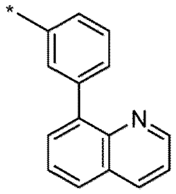
(B53),
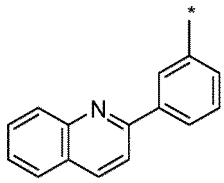
(B54),
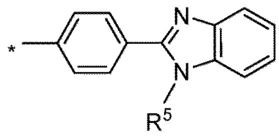
(B55),

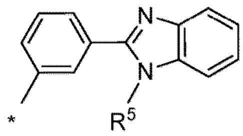
(B56),
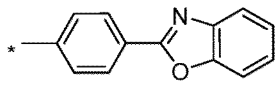
(B57),
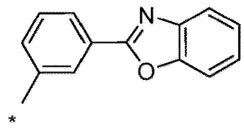
(B58),

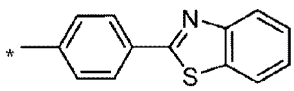
(B59),
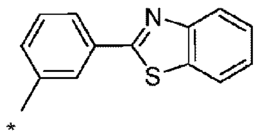
(B60),
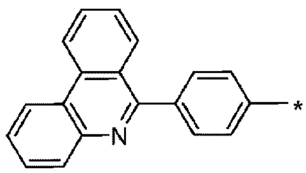
(B61),

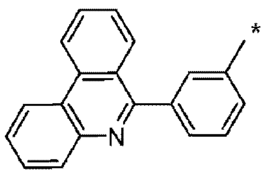
(B62);

oder

f) B63 bis B65 unsubstituierte anellierte Heteroarylengruppen sind:

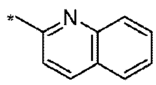
(B63),
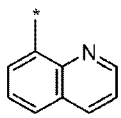
(B64)
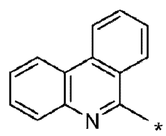
(B65); oder

i) B71 bis B77 Carbazolgruppen sind:

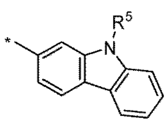
(B71),
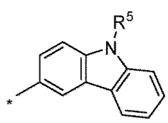
(B72),
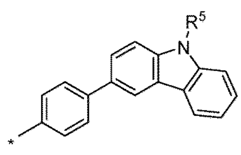
(B73),

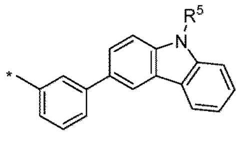
(B74),
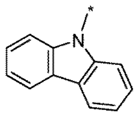
(B75),
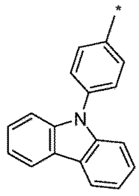
(B76),
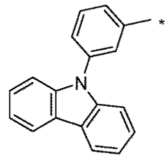
(B77);

wobei

der Substituent $R^5$ für H steht.

**9.** Organische Halbleiterschicht nach einem der Ansprüche 1 bis 8, wobei die Verbindung der Formel 1 aus D1 bis D8 ausgewählt ist:

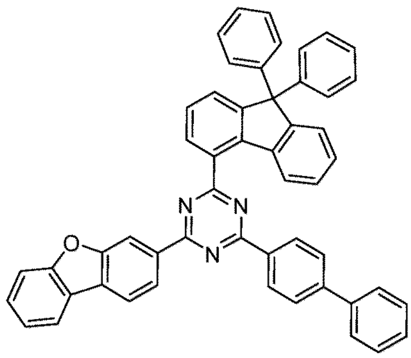
(D1),
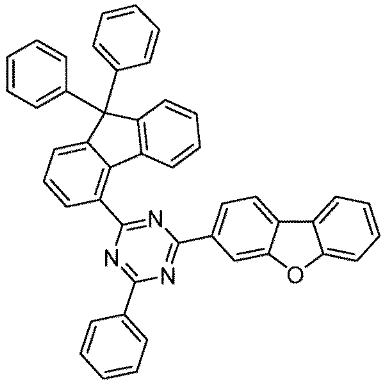
(D2),

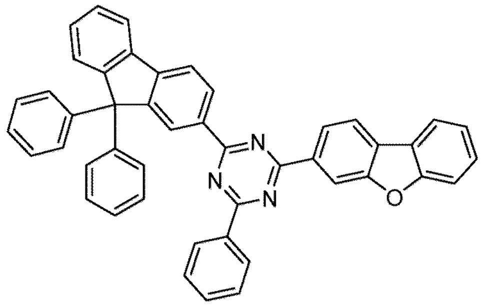
(D3),
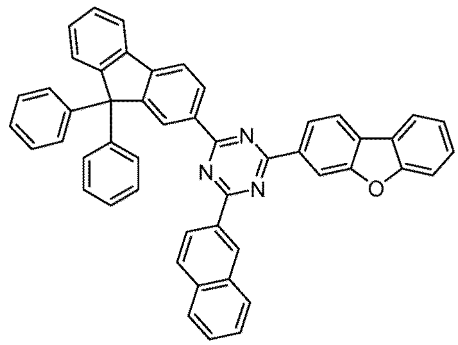
(D4),

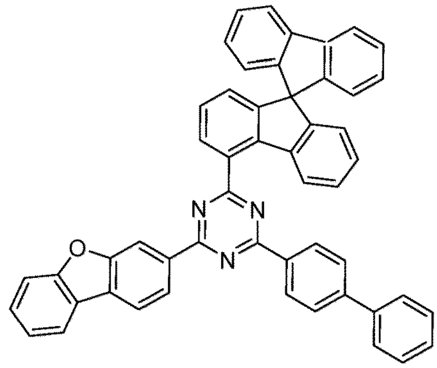

(D5),

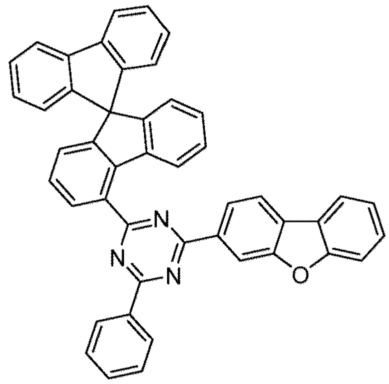

(D6),

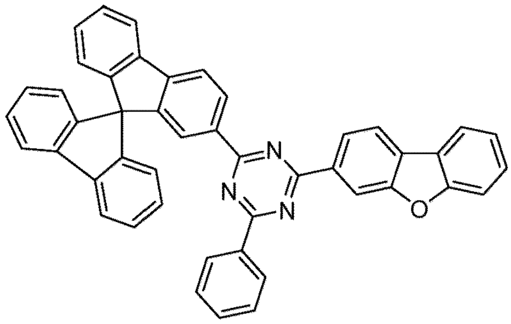

(D7),

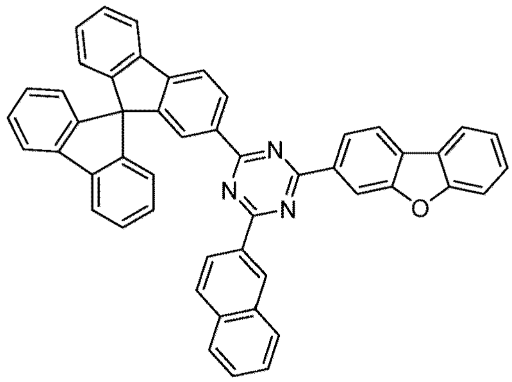

(D8).

**10.** Organische Halbleiterschicht, umfassend eine Verbindung der Formel 1:

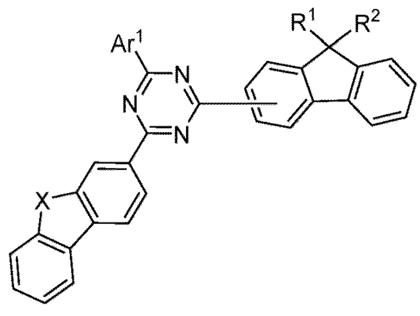

(1),

wobei

X für O, S oder Se steht;
$Ar^1$ aus $C_1$- bis $C_{16}$-Alkyl, substituiertem oder unsubstituiertem $C_6$- bis $C_{40}$-Aryl oder substituiertem oder unsubstituiertem $C_3$- bis $C_{40}$-Heteroaryl ausgewählt ist, wobei

die Substituenten des substituierten $C_6$-bis $C_{40}$-Aryls und substituierten $C_3$- bis $C_{40}$-Heteroaryls aus $C_1$- bis $C_{16}$-Alkyl, $C_1$-bis $C_{16}$-Alkoxy, teil- oder perfluoriertem $C_1$- bis $C_{16}$-Alkyl, teil- oder perfluoriertem $C_1$- bis $C_{16}$-Alkoxy, teil-oder perdeuteriertem $C_1$- bis $C_{16}$-Alkyl, verzweigtem $C_3$- bis $C_{16}$-Alkyl, cyclischem $C_3$- bis $C_{16}$-Alkyl, verzweigtem $C_3$- bis $C_{16}$-Alkoxy, cyclischem $C_3$- bis $C_{16}$-Alkoxy, teil- oder perdeuteriertem $C_1$- bis $C_{16}$-Alkoxy, $C_6$- bis $C_{24}$-Aryl, $C_3$- bis $C_{25}$-Heteroaryl, $-PXR^3R^4$, D, F oder CN ausgewählt sind, wobei $R^3$, $R^4$ unabhängig aus $C_1$- bis $C_{16}$-Alkyl, $C_1$-bis $C_{16}$-Alkoxy, teil- oder perfluoriertem $C_1$- bis $C_{16}$-Alkyl, teil- oder perfluoriertem $C_1$- bis $C_{16}$-Alkoxy, teil-oder perdeuteriertem $C_1$- bis $C_{16}$-Alkyl, teil- oder perdeuteriertem $C_1$- bis $C_{16}$-Alkoxy, $C_6$- bis $C_{18}$-Aryl, $C_3$- bis $C_{25}$-Heteroaryl ausgewählt sind,

wobei das substituierte $C_6$- bis $C_{40}$-Aryl und/oder substituierte $C_3$- bis $C_{40}$-Heteroaryl von $Ar^1$ mindestens einen-$PXR^3R^4$-Substituenten umfasst;

$R^1$, $R^2$ unabhängig aus substituiertem oder unsubstituiertem $C_6$- bis $C_{24}$-Aryl, substituiertem oder unsubstituiertem $C_3$- bis $C_{24}$-Heteroaryl ausgewählt sind oder gegebenenfalls $R^1$ und $R^2$ aus substituiertem oder unsubstituiertem $C_6$- bis $C_{24}$-Aryl und substituiertem oder unsubstituiertem $C_3$- bis $C_{24}$-Heteroaryl ausgewählt sind und über eine Einfachbindung so miteinander verknüpft sind, dass sich ein fünfgliedriger Ring ergibt,
wobei die Substituenten des substituierten $C_6$- bis $C_{24}$-Aryls und substituierten $C_3$- bis $C_{24}$-Heteroaryls aus H, D, $C_1$- bis $C_{16}$-Alkyl, teil- oder perfluoriertem $C_1$- bis $C_{16}$-Alkyl, teil-oder perdeuteriertem $C_1$- bis $C_{16}$-Alkyl, $C_1$-bis $C_{16}$-Alkoxy, $C_1$- bis $C_{16}$-Thioalkyl, verzweigtem $C_3$- bis $C_{16}$-Alkyl, cyclischem $C_3$- bis $C_{16}$-Alkyl, verzweigtem $C_3$- bis $C_{16}$-Alkoxy, cyclischem $C_3$- bis $C_{16}$-Alkoxy, verzweigtem $C_3$- bis $C_{16}$-Thioalkyl, cyclischem $C_3$- bis $C_{16}$-Thioalkyl, $C_6$- bis $C_{24}$-Aryl und $C_3$- bis $C_{25}$-Heteroaryl ausgewählt sind;

und

- einen Dotanden, der aus einem organischen Metallkomplex oder einem Metallhalogenid ausgewählt ist.

**11.** Organische Halbleiterschicht nach Anspruch 10, wobei $Ar^1$ unabhängig aus den Strukturen C1 bis C5 ausgewählt ist:

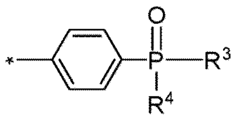
(C1),
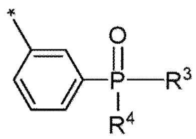
(C2),

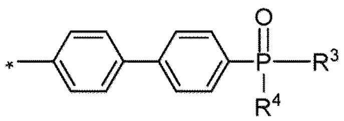
(C3),
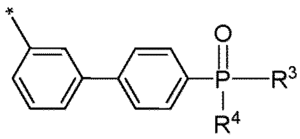
(C4),

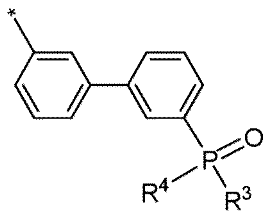
(C5),

wobei R3, $R^4$ unabhängig aus $C_1$- bis $C_{16}$-Alkyl, $C_1$- bis $C_{16}$-Alkoxy, teil- oder perfluoriertem $C_1$- bis $C_{16}$-Alkyl, teil- oder perfluoriertem $C_1$- bis $C_{16}$-Alkoxy, teil- oder perdeuteriertem $C_1$- bis $C_{16}$-Alkyl, teil- oder perdeuteriertem $C_1$- bis $C_{16}$-Alkoxy, $C_6$- bis $C_{18}$-Aryl, $C_3$- bis $C_{25}$-Heteroaryl ausgewählt sind, vorzugsweise $R^3$ und $R^4$ gleich gewählt sind.

**12.** Organische elektronische Vorrichtung, umfassend eine organische Halbleiterschicht nach einem der vorhergehenden Ansprüche 1 bis 11.

**13.** Organische elektronische Vorrichtung nach Anspruch 12, wobei die organische Halbleiterschicht zwischen einer Emissionsschicht und einer Kathodenschicht, vorzugsweise zwischen einer Hilfselektronentransportschicht und einer Kathodenschicht, weiter bevorzugt zwischen einer Hilfselektronentransportschicht und einer Elektroneninjektionsschicht, angeordnet ist.

**14.** Organische elektronische Vorrichtung nach Anspruch 12 oder 13, wobei es sich bei der elektronischen Vorrichtung

um eine lichtemittierende Vorrichtung, einen Dünnschichttransistor, eine Batterie, eine Anzeigevorrichtung oder eine Photovoltaikzelle und vorzugsweise eine lichtemittierende Vorrichtung handelt.

## Revendications

**1.** Couche de semi-conducteur organique comprenant :

- un composé de formule 1 :

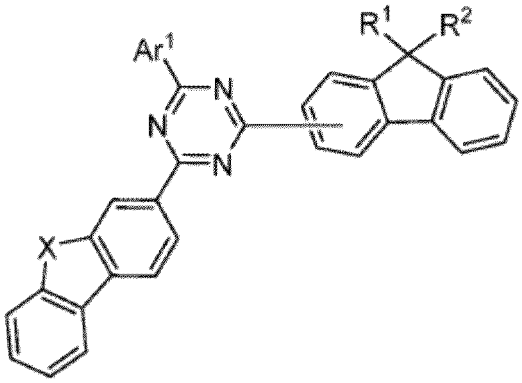

(1),

X étant O, S ou Se ;
$Ar^1$ étant choisi parmi $C_6$ à $C_{24}$ aryle non substitué ou $C_3$ à $C_{36}$ hétéroaryle non substitué ;
$R^1$, $R^2$ étant indépendamment choisis parmi $C_6$ à $C_{24}$ aryle substitué ou non substitué, $C_3$ à $C_{24}$ hétéroaryle substitué ou non substitué, ou $R^1$ et $R^2$ étant choisis parmi $C_6$ à $C_{24}$ aryle substitué ou non substitué et $C_3$ à $C_{24}$ hétéroaryle substitué ou non substitué et liés l'un à l'autre via une simple liaison de sorte qu'un cycle à cinq chaînons est formé,
les substituants des $C_6$ à $C_{24}$ aryle substitué et $C_3$ à $C_{24}$ hétéroarylène substitué étant choisis parmi H, D, $C_1$ à $C_{16}$ alkyle, $C_1$ à $C_{16}$ alkyle partiellement ou perfluoré, $C_1$ à $C_{16}$ alkyle partiellement ou perdeutéré, $C_1$ à $C_{16}$ alcoxy, $C_1$ à $C_{16}$ thioalkyle, $C_3$ à $C_{16}$ alkyle ramifié, $C_3$ à $C_{16}$ alkyle cyclique, $C_3$ à $C_{16}$ alcoxy ramifié, $C_3$ à $C_{16}$ alcoxy cyclique, $C_3$ à $C_{16}$ thioalkyle ramifié, $C_3$ à $C_{16}$ thioalkyle cyclique, $C_6$ à $C_{24}$ aryle et $C_3$ à $C_{25}$ hétéroaryle ;

et
- un dopant choisi parmi un complexe métallique organique ou un halogénure métallique.

**2.** Couche de semi-conducteur organique selon la revendication 1, le dopant étant non émissif.

**3.** Couche de semi-conducteur organique selon la revendication 1 ou 2, le dopant étant choisi parmi un complexe métallique monovalent ou divalent organique, préférablement un complexe d'alcali et/ou un halogénure d'alcali, préférablement le dopant étant choisi parmi LiQ ou le borate de lithium.

**4.** Couche de semi-conducteur organique selon l'une quelconque des revendications 1 à 3, X étant choisi parmi O ou S, préférablement O.

**5.** Couche de semi-conducteur organique selon l'une quelconque des revendications 1 à 4,
$R^1$, $R^2$ étant indépendamment choisis parmi $C_6$ à $C_{24}$ aryle substitué ou non substitué, $C_3$ à $C_{24}$ hétéroaryle substitué ou non substitué,
les substituants des $C_6$ à $C_{24}$ aryle substitué et $C_3$ à $C_{24}$ hétéroarylène substitué étant choisis parmi H, D, $C_1$ à $C_{16}$ alkyle, $C_1$ à $C_{16}$ alkyle partiellement ou perfluoré, $C_1$ à $C_{16}$ alkyle partiellement ou perdeutéré, $C_1$ à $C_{16}$ alcoxy, $C_1$ à $C_{16}$ thioalkyle, $C_3$ à $C_{16}$ alkyle ramifié, $C_3$ à $C_{16}$ alkyle cyclique, $C_3$ à $C_{16}$ alcoxy ramifié, $C_3$ à $C_{16}$ alcoxy cyclique, $C_3$ à $C_{16}$ thioalkyle ramifié, $C_3$ à $C_{16}$ thioalkyle cyclique, $C_6$ à $C_{24}$ aryle et $C_3$ à $C_{25}$ hétéroaryle.

**6.** Couche de semi-conducteur organique selon l'une quelconque des revendications 1 à 5,
$R^1$, $R^2$ étant indépendamment choisis parmi $C_6$ à $C_{24}$ aryle non substitué, ou $C_3$ à $C_{24}$ hétéroaryle non substitué.

**7.** Couche de semi-conducteur organique selon l'une quelconque des revendications 1 à 6, $Ar^1$ étant choisi parmi $C_6$ à $C_{12}$ aryle non substitué, ou $C_3$ à $C_{18}$ hétéroaryle non substitué, préférablement phényle ou biphényle et de plus de manière préférée phényle.

**8.** Couche de semi-conducteur organique selon l'une quelconque des revendications 1 à 7, $Ar^1$ étant indépendamment choisi parmi B1 à B65 et B71 à B77 :

a) B1 à B6 étant des groupes non hétéroaryle non substitués :

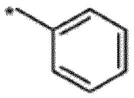
(B1),
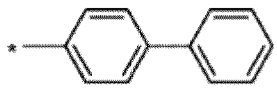
(B2),
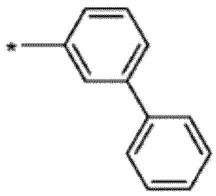
(B3),
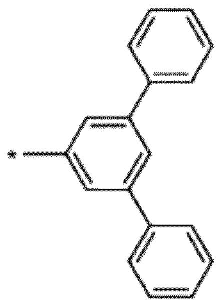
(B4),

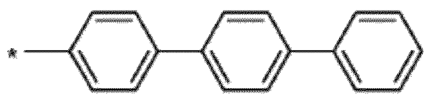
(B5),
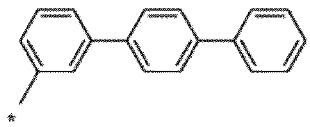
(B6),

ou

b) B7 à B23 étant des groupes non hétéroaryle annelés non substitués :

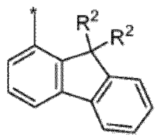
(B7),
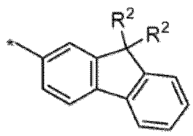
(B8),
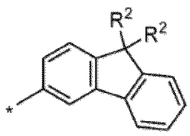
(B9),
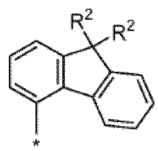
(B10),
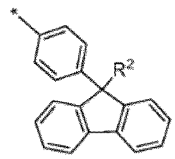
(B11),

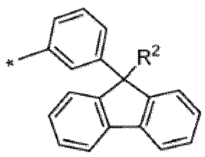
(B12),
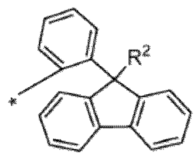
(B13),
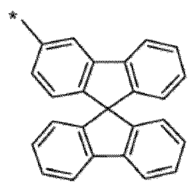
(B14),
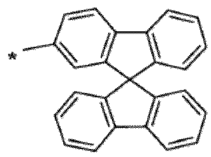
(B15),

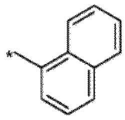 (B16), 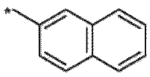 (B17), 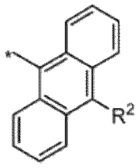 (B18), 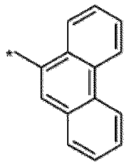 (B19), 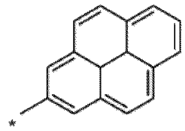 (B20),

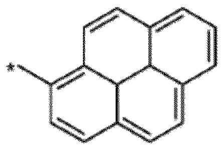 (B21), 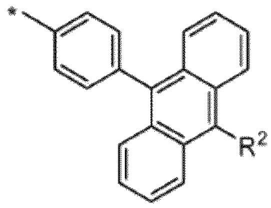 (B22), 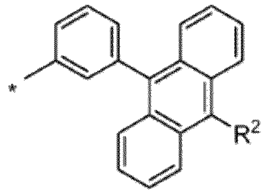 (B23) ;

ou

c) B24 à B31 étant un groupe dibenzofurane/dibenzothiophène :

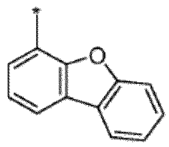 (B24), 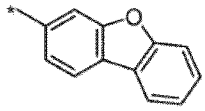 (B25), 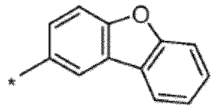 (B26), 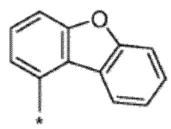 (B27),

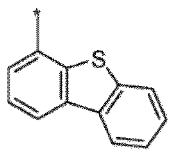 (B28), 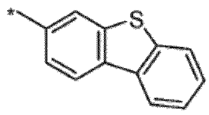 (B29), 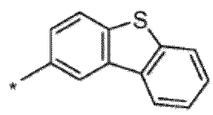 (B30),

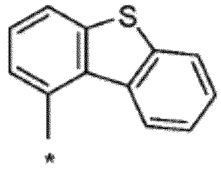 (B31) ;

ou

d) B32 à B34 étant des groupes pyridine non substitués :

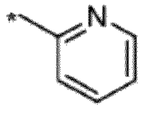 (B32), 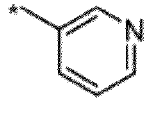 (B33), 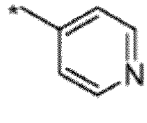 (B34);

ou

e) B35 à B62 étant des groupes hétéroarylène non substitués :

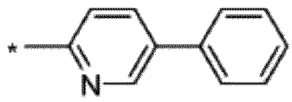

(B35),

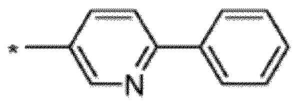

(B36),

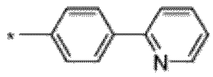

(B37),

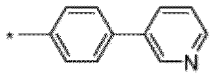

(B38),

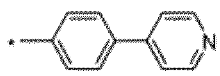

(B39),

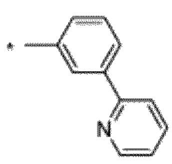

(B40),

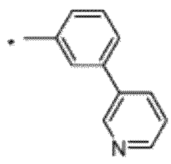

(B41),

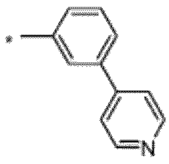

(B42),

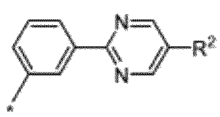

(B43),

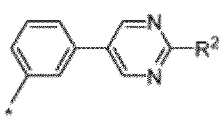

(B44),

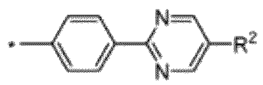

(B45),

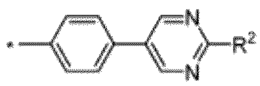

(B46),

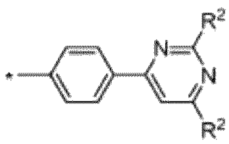

(B47),

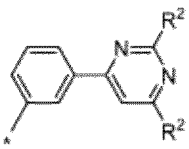

(B48),

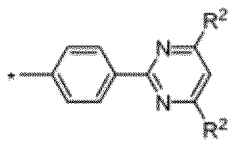

(B49),

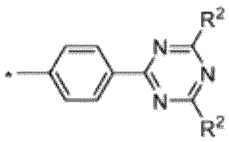

(B50),

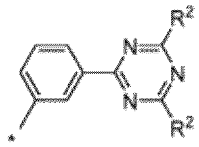

(B51),

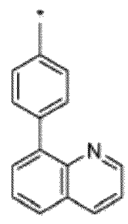

(B52),

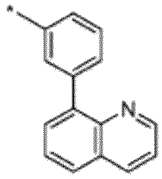

(B53),

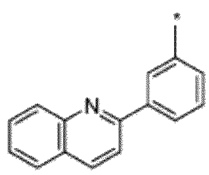

(B54),

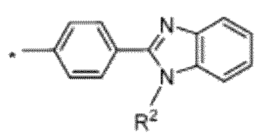

(B55),

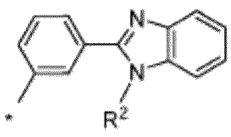
(B56),

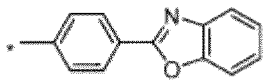
(B57),

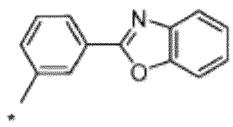
(B58),

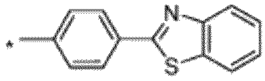
(B59),

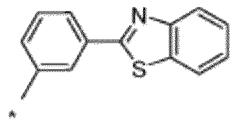
(B60),

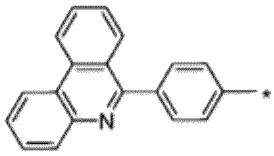
(B61),

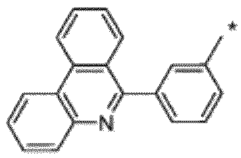
(B62) ;

ou

f) B63 à B65 étant des groupes hétéroarylène annelés non substitués :

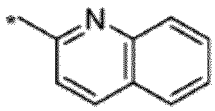
(B63),

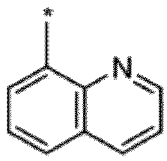
(B64)

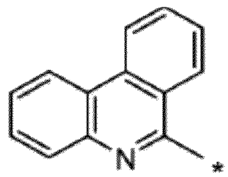
(B65) ;

ou

i) B71 à B77 étant des groupes carbazole

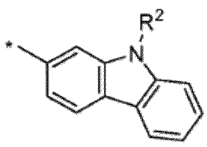
(B71),

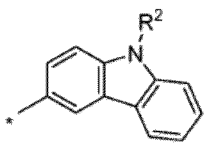
(B72),

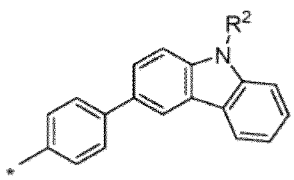
(B73),

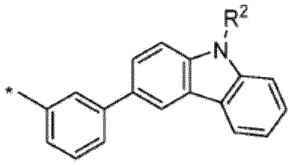
(B74),
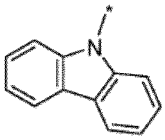
(B75),
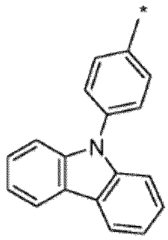
(B76),
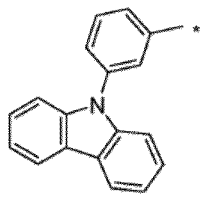
(B77);

le substituant $R^5$ étant H.

**9.** Couche de semi-conducteur organique selon l'une quelconque des revendications 1 à 8, le composé de formule 1 étant choisi parmi D1 à D8 :

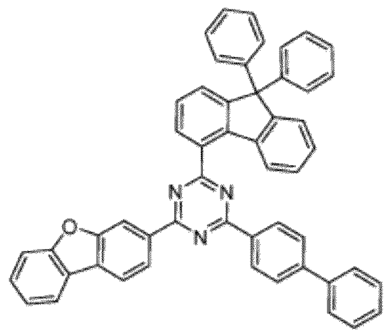
(D1),
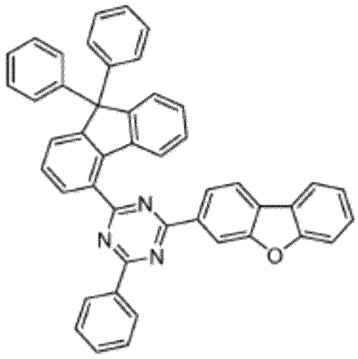
(D2),

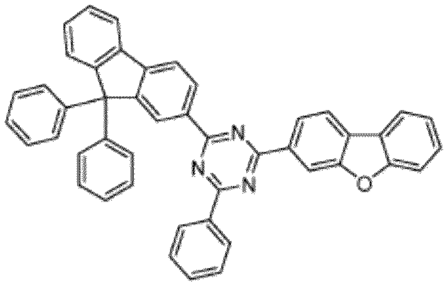
(D3),
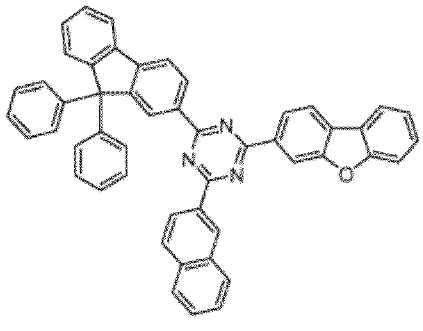
(D4),

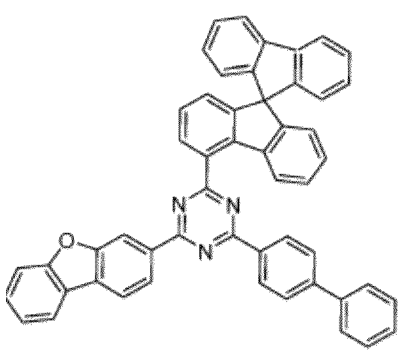
(D5),
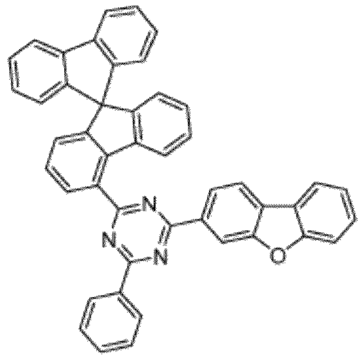
(D6),

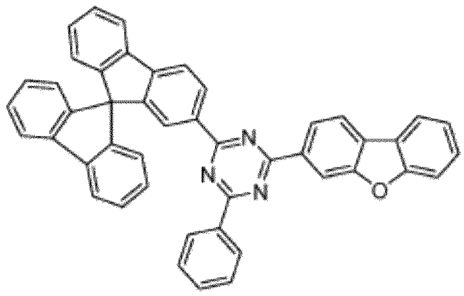

(D7),

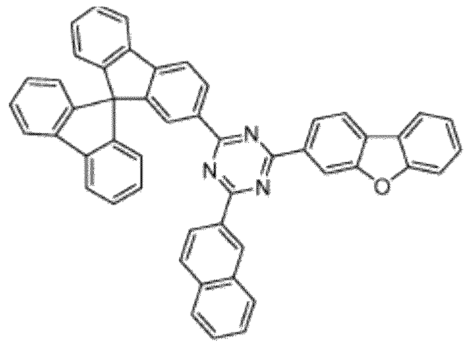

(D8).

.

**10.** Couche de semi-conducteur organique comprenant un composé de formule 1 :

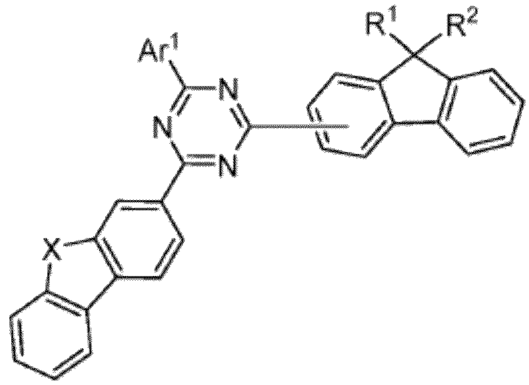

(1),

X étant O, S ou Se ;
$Ar^1$ étant choisi parmi $C_1$ à $C_{16}$ alkyle, $C_6$ à $C_{40}$ aryle substitué ou non substitué, $C_3$ à $C_{40}$ hétéroaryle substitué ou non substitué,
les substituants des $C_6$ à $C_{40}$ aryle substitué et $C_3$ à $C_{40}$ hétéroaryle substitué étant choisis parmi $C_1$ à $C_{16}$ alkyle, $C_1$ à $C_{16}$ alcoxy, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_3$ à $C_{16}$ alkyle ramifié, $C_3$ à $C_{16}$ alkyle cyclique, $C_3$ à $C_{16}$ alcoxy ramifié, $C_3$ à $C_{16}$ alcoxy cyclique, $C_1$ à $C_{16}$ alcoxy partiellement ou perdeutéré, $C_6$ à $C_{24}$ aryle, $C_3$ à $C_{25}$ hétéroaryle, $-PXR^3R^4$, D, F ou CN,

R3, $R^4$ étant indépendamment choisis parmi $C_1$ à $C_{16}$ alkyle, $C_1$ à $C_{16}$ alcoxy, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_1$ à $C_{16}$ alcoxy partiellement ou perdeutéré, $C_6$ à $C_{18}$ aryle, $C_3$ à $C_{25}$ hétéroaryle,
les $C_6$ à $C_{40}$ aryle substitué et/ou $C_3$ à $C_{40}$ hétéroaryle substitué de $Ar^1$ comprenant au moins un substituant $-PXR^3R^4$ ;

$R^1$, $R^2$ étant indépendamment choisis parmi $C_6$ à $C_{24}$ aryle substitué ou non substitué, $C_3$ à $C_{24}$ hétéroaryle substitué ou non substitué, ou éventuellement $R^1$ et $R^2$ étant choisis parmi $C_6$ à $C_{24}$ aryle substitué ou non substitué et $C_3$ à $C_{24}$ hétéroaryle substitué ou non substitué et liés l'un à l'autre via une simple liaison de sorte qu'un cycle à cinq chaînons est formé,
les substituants des $C_6$ à $C_{24}$ aryle substitué et $C_3$ à $C_{24}$ hétéroarylène substitué étant choisis parmi H, D, $C_1$ à $C_{16}$ alkyle, $C_1$ à $C_{16}$ alkyle partiellement ou perfluoré, $C_1$ à $C_{16}$ alkyle partiellement ou perdeutéré, $C_1$ à $C_{16}$ alcoxy, $C_1$ à $C_{16}$ thioalkyle, $C_3$ à $C_{16}$ alkyle ramifié, $C_3$ à $C_{16}$ alkyle cyclique, $C_3$ à $C_{16}$ alcoxy ramifié, $C_3$ à $C_{16}$ alcoxy cyclique, $C_3$ à $C_{16}$ thioalkyle ramifié, $C_3$ à $C_{16}$ thioalkyle cyclique, $C_6$ à $C_{24}$ aryle et $C_3$ à $C_{25}$ hétéroaryle ;

et

- un dopant choisi parmi un complexe métallique organique ou un halogénure métallique.

**11.** Couche de semi-conducteur organique selon la revendication 10, $Ar^1$ étant indépendamment choisi parmi les structures $C_1$ à C5 :

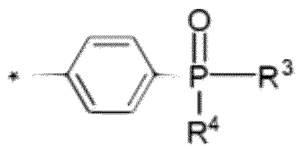
(C1),

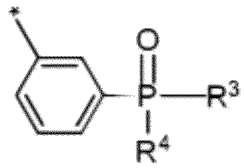
(C2),

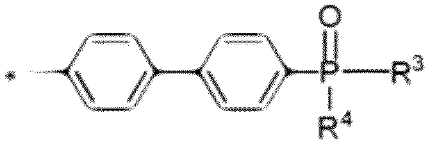
(C3),

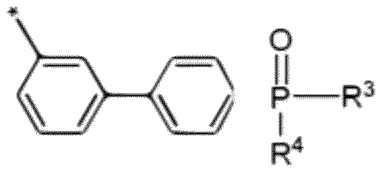
(C4),

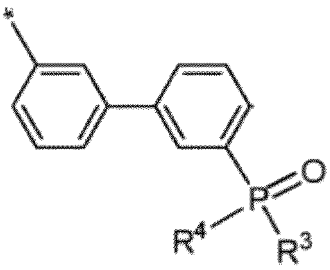
(C5),

R3, $R^4$ étant indépendamment choisis parmi $C_1$ à $C_{16}$ alkyle, $C_1$ à $C_{16}$ alcoxy, $C_1$ à $C_{16}$ alkyle partiellement ou perfluoré, $C_1$ à $C_{16}$ alcoxy partiellement ou perfluoré, $C_1$ à $C_{16}$ alkyle partiellement ou perdeutéré, $C_1$ à $C_{16}$ alcoxy partiellement ou perdeutéré, $C_6$ à $C_{18}$ aryle, $C_3$ à $C_{25}$ hétéroaryle, préférablement $R^3$, $R^4$ étant les mêmes.

**12.** Dispositif électronique organique comprenant une couche de semi-conducteur organique selon l'une quelconque des revendications précédentes 1 à 11.

**13.** Dispositif électronique organique selon la revendication 12, la couche de semi-conducteur organique étant agencée entre une couche d'émission et une couche de cathode, préférablement entre une couche de transport d'électrons auxiliaire et une couche de cathode, de manière plus préférée entre une couche de transport d'électrons auxiliaire et une couche d'injection d'électrons.

**14.** Dispositif électronique organique selon la revendication 12 ou 13, le dispositif électronique étant un dispositif émetteur de lumière, un transistor en film mince, une batterie, un dispositif d'affichage ou une cellule photovoltaïque, et préférablement un dispositif émetteur de lumière.

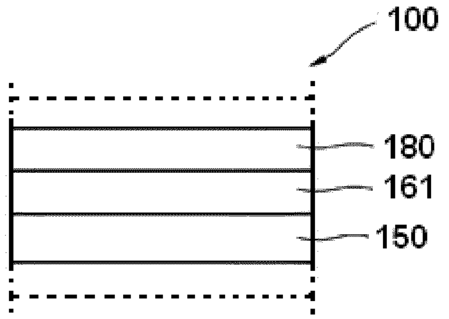


FIG. 1

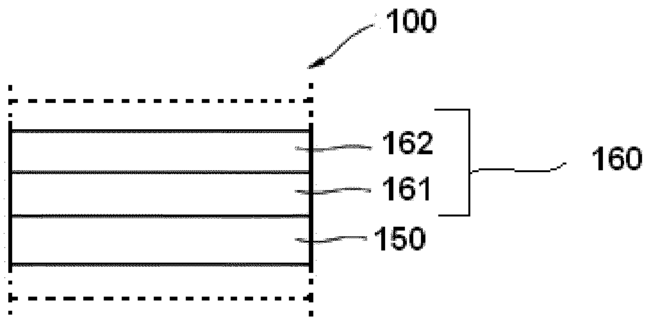


FIG. 2

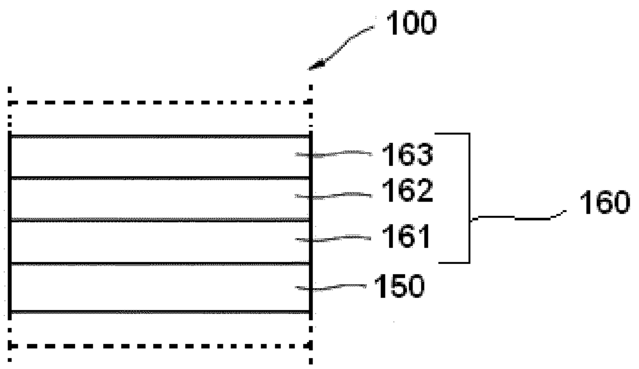


FIG. 3

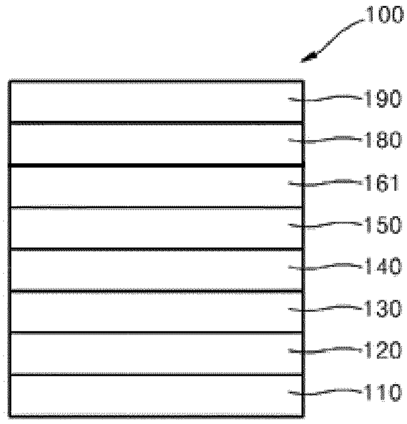


FIG. 4

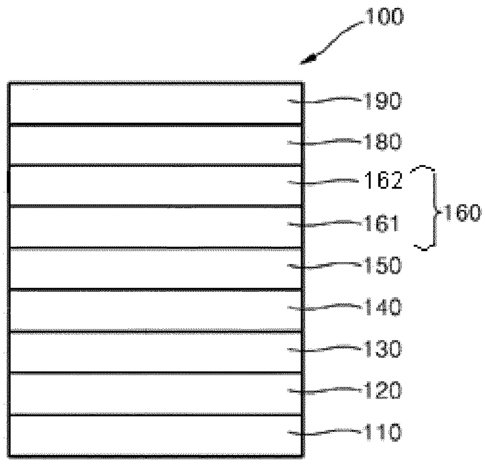


FIG. 5

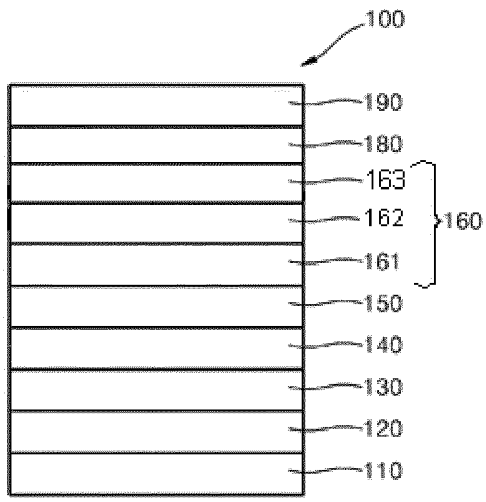


FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105153130 A
- US 6140763 A
- US 6614176 B
- US 2016248022 A
- WO 2013079217 A1
- WO 2013079676 A1
- WO 2013079678 A1
- JP 2008195623 B
- JP 2001291593 B
- US 20030165711 A
- KR 101537500
- KR 101537499
- KR 20170140115


### Non-patent literature cited in the description


- *CHEMICAL ABSTRACTS,* 1242056-42-3
- *CHEMICAL ABSTRACTS,* 1198399-61-9